# EUROPEAN PATENT APPLICATION

(11) **EP 4 834 350 A1**
(43) Date of publication of application: **04.11.2026**
(21) Application number: 24910335.9
(22) Date of filing: 08.11.2024
(51) Int. Cl.: A61K 31/7048, A61K 31/4375, A61P 11/00

(54) **PHARMACEUTICAL COMPOSITION FOR TREATING PULMONARY INFLAMMATORY DISEASE, PREPARATION THEREOF, AND USE THEREOF**

(30) Priority: 28.12.2023 CN 202311837477
(71) Applicant: Shandong University, Jinan, Shandong 250061 (CN); Jiuhua Huayuan Pharmaceutical Co., Ltd., Anhui 239000 (CN)
(72) Inventor: SHEN, Tao, Jinan, Shandong 250100 (CN); GUAN, Yueqin, Anhui 239004 (CN); XU, Zhenpeng, Jinan, Shandong 250100 (CN); PENG, Guangcheng, Jinan, Shandong 250100 (CN); LIU, Mingjie, Jinan, Shandong 250100 (CN); LI, Guohui, Jinan, Shandong 250100 (CN)
(74) Representative: Viering, Jentschura & Partner mbB Patent- und Rechtsanwälte
(86) International application number: PCT/CN2024/130814
(87) International publication number: WO 2025/139398

(57) **Abstract**

This application relates to the technical field of pharmaceutical compositions for pneumonia, and more specifically to a pharmaceutical composition for treating pulmonary inflammatory disease and a preparation and an application thereof. The provided pharmaceutical composition comprises the following components: 1 to 100 parts of a Bairuisu component and 1 to 20 parts of a matrine component; wherein the Bairuisu components are selected from kaempferol-3-O-glucorhamnoside, astragalin, kaempferol, Thesiuside A, Thesiuside B, or a combination thereof. The matrine component is selected from matrine, sophocarpine, Thesiumine A, Thesiumine B, or a combination thereof. The Bairuisu component exerts an attenuating effect on the toxicity of high-concentration matrine component. The Bairuisu components can inhibit the hepatotoxicity and nephrotoxicity of the matrine component and suppress the production of reactive oxygen species (ROS) induced by the matrine component.

## Description

This application claims the priority of Chinese Patent Application No. 2023118374770, filed with the CNIPA on Dec. 28, 2023, and titled "PHARMACEUTICAL COMPOSITION FOR TREATING PULMONARY INFLAMMATORY DISEASE, AND PREPARATION AND APPLICATION THEREOF", the entire contents of which are incorporated herein by reference.

### TECHNICAL FIELD

This application relates to the technical field of pharmaceutical compositions for pneumonia, and more specifically to a pharmaceutical composition for treating pulmonary inflammatory disease and a preparation and an application thereof.

### BACKGROUND

Pulmonary inflammatory diseases, including chronic obstructive pulmonary disease (COPD) and pneumonia, have high incidence rates and cause significant harm, and their pathogenesis is closely associated with inflammatory responses. For example, COPD has a high incidence and mortality rate worldwide, and the elderly population is at high risk for COPD. COPD is a preventable and treatable disease characterized by persistent airflow limitation, which is associated with chronic inflammation and autoimmune responses triggered by pathogenic microorganisms, cigarette smoke, toxic particles, or gases. Oxidative stress induced by harmful substances can stimulate alveolar macrophages to secrete various cytokines, such as interleukin-8 (IL-8) and tumor necrosis factor-α (TNF-α), promoting the release of proteases, increasing epithelial permeability, and elevating levels of lipid peroxides. These factors lead to the retention and activation of neutrophils in the airways and lungs, resulting in a significant increase in the release of inflammatory mediators, which further exacerbates the inflammatory response in the airways, lungs, and the whole body. Respiratory diseases are closely related to inflammatory responses; therefore, developing drugs for respiratory diseases from the perspective of treating inflammation is an effective strategy.

Acute or chronic inflammatory diseases can lead to the production of large amounts of nitric oxide (NO) in the body. Persistently high levels of NO in the body can directly damage DNA and proteins, thereby causing tissue damage. Thus, NO production inhibitory activity can reflect a compound's anti-inflammatory activity. Quinone reductase (QR) is a phase II detoxification enzyme that catalyzes the reduction of quinones and eliminates reactive oxygen species in the body. It is generally believed that phase II detoxification enzymes are mainly responsible for the metabolic detoxification of harmful oxidants and chemical carcinogens. Therefore, QR-inducing activity can reflect a compound's inhibitory effect on inflammatory responses and oxidative stress, as well as its preventive effect against inflammation-related diseases such as COPD and pneumonia. Studies have shown that oxidative stress and inflammatory responses can interact with each other: oxidative stress can induce inflammatory responses, and inflammatory responses can also exacerbate oxidative stress. Persistent oxidative stress and inflammatory responses lead to the massive release of inflammatory mediators, changes in cell permeability, necrosis, etc. Moreover, increased ROS levels cause damage to biological macromolecules such as DNA, proteins, and lipids, which can trigger a series of inflammatory diseases.

The formation of the NLRP3 inflammasome activates procaspase-1, which in turn generates caspase-1, promoting the maturation and secretion of the inflammatory cytokine IL-1β. Many serious human diseases, such as COPD, asthma, allergic airway inflammation, pneumonia, diabetic nephropathy, gout, non-alcoholic steatohepatitis, myocardial infarction, hypertension, and Alzheimer's disease, are closely associated with excessive activation of the NLRP3 inflammasome. Therefore, it is of great significance to develop drugs that inhibit NLRP3 inflammasome activation.

Ferroptosis is a distinct form of programmed cell death that is closely related to the occurrence and development of many lung diseases, including acute lung injury, chronic obstructive pulmonary disease, asthma, cystic fibrosis, pulmonary infections (bacterial, viral, and fungal), and vascular lung diseases (ischemia-reperfusion injury and pulmonary arterial hypertension). Ferroptosis is mainly characterized by intracellular iron accumulation, depletion of glutathione, inactivation of glutathione peroxidase, and promotion of lipid peroxidation. Ferroptosis can be regulated by a variety of signaling pathways, including p62 Kelch-like ECH-associated protein 1 (Keap1)-nuclear factor erythroid 2-related factor 2 (Nrf2)/solute carrier family 7 member 11 (SLC7A11)/glutathione peroxidase 4 (GPX4) and acyl-CoA synthetase long-chain family member 4 (ACSL4). Many interventions, including ferroptosis inducers and inhibitors, iron chelators, lipid peroxidation inhibitors, and antioxidants, have been introduced for ferroptosis-related lung diseases.

T-cell-mediated pulmonary inflammatory responses have been shown to play a key role in lung diseases. The differentiation of CD4 T lymphocytes proceeds from naïve CD4 cells to mature CD4 cells, which further differentiate into different cell subsets, including Th1, Th2, Th17, and regulatory Treg cells. Different subsets secrete different cytokines and chemokines, which mediate (or inhibit) the recruitment of inflammatory cells to the site of infection, thereby promoting (or inhibiting) the progression of chronic lung diseases, such as chronic obstructive pulmonary disease (COPD), lung cancer, cystic fibrosis, and asthma. There is close crosstalk between pulmonary immunity and the gut microbiota, which can be mediated through the lymphatic and circulatory systems. In COPD, exogenous particulate matter or cigarette smoke can cause intestinal inflammatory responses and alterations in the composition of the gut microbiota. Furthermore, components derived from the gut microbiota or their metabolites can damage the intestinal barrier, leading to local and systemic immune responses, which in turn affect the immune response and pathological damage in the lungs. The lung-gut interaction is bidirectional. The lungs can also affect the gut through lymphocyte migration and inflammatory cytokines.

*Thesium chinense* Turcz. (known as Bai Rui Cao in Chinese) is a plant of the genus *Thesium* in the family Santalaceae; the whole plant is used as medicine. It is pungent, slightly bitter, and cold in nature, and acts on the Spleen, Kidney, and Lung meridians. It has the functions of clearing heat, detoxifying, tonifying the kidney, and astringing essence. Research on the chemical composition of *Thesium chinense* is relatively weak. The literature has reported more than 60 compounds, including flavonoids, alkaloids, organic acids, and terpenoids. The material basis and mechanism of action of *Thesium chinense* in treating inflammatory diseases remain unclear.

The Bairuisu component mainly includes kaempferol-3-O-glucorhamnoside (Bairuisu I), astragalin (Bairuisu II), and kaempferol (Bairuisu III). According to literature reports, kaempferol-3-O-glucorhamnoside has anti-inflammatory and antidiabetic activities. Astragalin has anti-inflammatory, antioxidant, neuroprotective, anti-osteoporotic, and anticancer effects. Kaempferol has anti-inflammatory, antioxidant, anti-osteoporotic, and anticancer effects. Thus, the Bairuisu component has broad application prospects in the field of new drug development. Kaempferol-3-O-glucorhamnoside, astragalin, and kaempferol are the main components of *Thesium chinense*, but their purification is difficult. To date, there have been no reports on the large-scale preparation of pure kaempferol-3-O-glucorhamnoside, astragalin, and kaempferol from *Thesium chinense*. Meanwhile, the Bairuisu component has poor water solubility and low bioavailability. Previous literature has only identified three alkaloids from *Thesium chinense*, and their contents are extremely low; among them, sophocarpine is a matrine component.

The matrine component belongs to the quinolizidine alkaloids, whose molecular skeleton is formed by the fusion of two quinolizidine rings. This class of alkaloids is widely present in the leguminous plants *Sophora flavescens*, *Sophora alopecuroides*, and *Sophora tonkinensis*. Representative compounds include matrine, sophoridine, sophocarpine, sophoramine, and sophoranol. Matrine has a variety of pharmacological activities, such as anti-tumor, anti-arrhythmic, anti-diabetic, immunosuppressive, anti-inflammatory, antibacterial, antiviral, antiparasitic, anti-fibrotic, and neuroprotective effects. Matrine-type alkaloids are used in clinical practice due to their broad pharmacological activities; however, their considerable toxicity limits their development as therapeutic agents, especially because long-term use can cause liver and kidney damage.

### SUMMARY

It is an objective of embodiments of the present application to provide a pharmaceutical composition for treating pulmonary inflammatory disease and a preparation and an application thereof, in order to address the technical problem in the existing technology that there is no drug capable of effectively treating pulmonary inflammatory diseases.

### TECHNICAL SOLUTIONS

Embodiments of the present application adopt the following technical solution:

In a first aspect, the present application provides a pharmaceutical composition for treating pulmonary inflammatory disease, comprising the following components:
1 to 100 parts of a Bairuisu component, and
1 to 20 parts of a matrine component.

The Bairuisu component has a structural formula as follows: 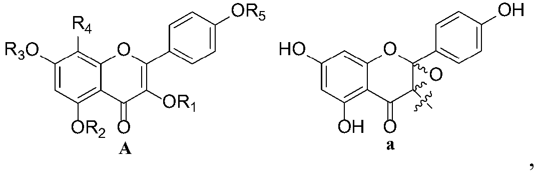 in which, R₄ represents H or group a; R₁, R₂, R₃, and R₅ are independently selected from one of the following groups: H, CH₃, CH₃CO, glucopyranosyl, rhamnopyranosyl, glucorhamnosyl, diglucosyl, xylopyranosyl, sucrosyl.

The matrine component has a structural formula as follows: 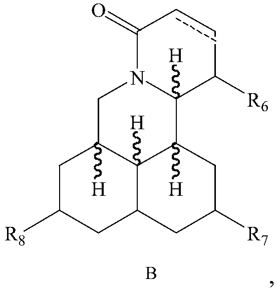 in which, R₆, R₇, and R₈ are independently selected from one of the following groups: H, OH, OCH₃, or CH₃COO.

In some embodiments, the pharmaceutical composition comprises the following components:
60 to 66 parts of the Bairuisu component, and
2 to 5 parts of the matrine component.

In some embodiments, the Bairuisu component is selected from at least one of kaempferol-3-O-glucorhamnoside, astragalin, kaempferol, Thesiuside A, and Thesiuside B.

In some embodiments, the matrine component is selected from at least one of matrine, sophocarpine, Thesiumine A, and Thesiumine B.

In some embodiments, the pharmaceutical composition comprises the following components:
10 to 20 parts of Thesiuside A,
10 to 20 parts of Thesiuside B,
1 to 2 parts of Thesiumine A, and
1 to 2 parts of Thesiumine B.

In some embodiments, the pharmaceutical composition comprises the following components:
10 to 15 parts of kaempferol, and
1 part of matrine.

In some embodiments, an extraction method of the Thesiuside A and the Thesiuside B comprises the following steps:
crushing whole herbs of dried *Thesium chinense* Turcz., soaking at 20°C to 40°C for 1 hr to 10 hrs, followed by extraction by heating under reflux with 60% to 80% ethanol to obtain an extract solution; filtering and concentrating the extract solution in sequence to obtain a total crude extract;
suspending the total crude extract in water, then sequentially extracting with petroleum ether, dichloromethane and ethyl acetate to obtain organic extract solutions;
subjecting an ethyl acetate extract solution to a normal-phase silica gel column chromatography, in which, a gradient elution is performed first with a petroleum ether/ethyl acetate eluent in a volume ratio of (0.1 to 1) : (0.1 to 1), and then with an ethyl acetate/methanol eluent in a volume ratio of (0.1 to 1) : (0.1 to 1); collecting eight fractions in chronological order to obtain first elution fractions E1 to E8;
subjecting a first elution fraction E7 to an MCI column to remove pigments, performing gradient elution with a methanol/water eluent in a volume ratio of (1 to 3) : ( 0.1 to 7), collecting eight fractions in chronological order to obtain second elution fractions E7a to E7h;
subjecting a second elution fraction E7d to normal-phase silica gel column chromatography, performing gradient elution with a dichloromethane/methanol eluent in a volume ratio of (0.1 to 30) : 1, collecting eight fractions in chronological order to obtain third elution fractions E7a to E7h; and
subjecting a third elution fraction E7d5 to separation and purification using Sephadex LH-20 and C18 reverse-phase silica gel to obtain the compounds Thesiuside A and Thesiuside B.

Thesiuside A has a structural formula as follows: 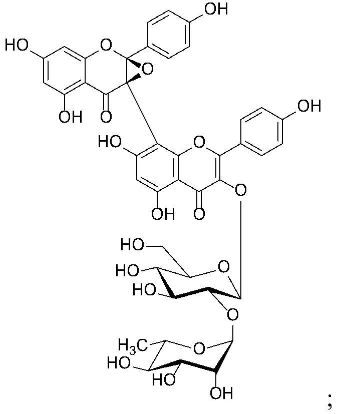 and
Thesiuside B has a structural formula as follows:

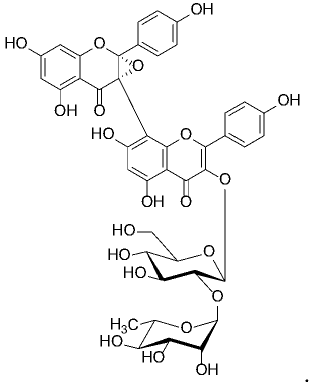

In some embodiments, an extraction method of Thesiumine A and Thesiumine B comprises the following steps:
crushing whole herbs of dried *Thesium chinense* Turcz., extracting with an aqueous hydrochloric acid solution having a mass concentration of 0.1 mol/L to 0.5 mol/L, adjusting a resulting extract solution to an alkaline pH, repeatedly extracting with dichloromethane, and concentrating under reduced pressure to obtain a total alkaloid extract;
subjecting the total alkaloid extract to a normal-phase silica gel column chromatography, eluting with a petroleum ether/dichloromethane/ammonia water eluent in a volume ratio of 1: 1: (0.02 to 0.03); performing gradient elution with a dichloromethane/methanol eluent in a volume ratio of (6 to 97): (3 to 4), collecting 10 fractions in chronological order to obtain fourth elution fractions S1 to S10;
purifying a fourth elution fraction S5 by Sephadex LH-20 column chromatography using methanol as an eluent, subjecting a purified S5 fraction to HPLC separation using a mobile phase having a pH of 3 to 4 and a methanol volume percentage of 5% to 10%; and
removing impurities through a macroporous adsorption resin column to obtain Thesiumine B and Thesiumine A.

Thesiumine A has a structural formula as follows: 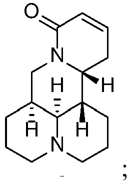 and
Thesiumine B has a structural formula as follows:

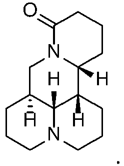

In some embodiments, the extraction process for kaempferol-3-O-glucorhamnoside, astragalin, and kaempferol comprises the following steps:
crushing whole herbs of dried *Thesium chinense* Turcz., soaking at 20°C to 40°C for 1 hr to 10 hrs, followed by extraction by heating under reflux with an aqueous ethanol solution having a mass percentage of 60% to 80% to obtain an extract solution; filtering and concentrating the extract solution in sequence to obtain a total crude extract;
suspending the total crude extract in water, then sequentially extracting with petroleum ether, dichloromethane and ethyl acetate to obtain organic extract solutions;
diluting an ethyl acetate extract solution with water, passing a resulting mixture through an MCI column, and then eluting sequentially with aqueous methanol solutions having mass percentages of 20 to 30%, 40 to 50%, and 60 to 70%, yielding a first fraction, a second fraction, and a third fraction, respectively;
further eluting the first fraction sequentially with pure water, aqueous ethanol solutions having mass percentages of 20% to 30%, 40% to 50%, and 60% to 70%, and pure ethanol, yielding a fraction a, a fraction b, a fraction c, a fraction d, and a fraction e, respectively;
purifying the fraction b by gel column chromatography using methanol as an eluent, followed by recrystallization to obtain kaempferol-3-O-glucorhamnoside;
purifying the fraction c by gel column chromatography using methanol as the eluent, followed by recrystallization to obtain astragalin; and
purifying the third fraction by gel column chromatography using methanol as the eluent, and followed by recrystallization to obtain kaempferol.

A second aspect of the present application provides a preparation for treating pulmonary inflammatory diseases, comprising: the aforementioned pharmaceutical composition as the active ingredient, having a mass percentage in the preparation of 0.1% to 99.9%, with a remainder being pharmaceutically acceptable excipients. A dosage form of the preparation is a tablet, sugar-coated tablet, film-coated tablet, enteric-coated tablet, capsule, oral solution, buccal tablet, granules, oral granules, pill, powder, ointment, pellet, suspension, fine powder, injection, suppository, cream, spray, drop, or patch.

A third aspect of the present application provides an application of the aforementioned pharmaceutical composition for treating the pulmonary inflammatory diseases in the manufacturing of a medicament for treating chronic obstructive pulmonary disease, acute lung injury, or lower respiratory tract infections.

### BENEFICIAL EFFECTS

Advantages of the embodiments of the first aspect of the present application are summarized as follows: the pharmaceutical composition provided by the present application mainly comprises a Bairuisu component and a matrine component. The Bairuisu component has QR-inducing activity, is capable of inhibiting the excessive production of NO in LPS-induced RAW264.7 mouse macrophages, alleviating LPS-induced pathological changes in mouse lung tissue, inhibiting the LPS-induced increase of the inflammatory cytokine IL-1β in mouse bronchoalveolar lavage fluid, and reducing the number of inflammatory cells in mouse blood, thereby exerting a lung-protective effect and alleviating pulmonary inflammation. The Bairuisu component and the matrine component have a synergistic effect, and they exert therapeutic effects on pulmonary diseases through different mechanisms. Specifically, the Bairuisu component acts through a novel mechanism of regulating ferroptosis-related pathway proteins SLC7A11 and GPX4 to treat acute lung injury, while the matrine component can inhibit the excessive activation of the NLRP3 inflammasome and suppress NF-κB activation. The two components synergistically exert a lung-protective effect through different mechanisms. Moreover, the binding of the Bairuisu component and the matrine component to key inflammation-regulating proteins provides better anti-inflammatory effects. Therefore, the two components have an interaction characterized by "synergistic efficacy, reduced toxicity, complementary mechanisms, and mutual solubilization," which is conducive to widespread application.

Advantages of the embodiments of the second aspect of the present application are summarized as follows: in the provided preparation for treating pulmonary inflammatory diseases, the aforementioned pharmaceutical composition constitutes 0.1% to 99.9% by mass of the composition as an active ingredient; thus, the provided preparation alleviates pulmonary inflammation, protects the lungs, exhibits excellent anti-inflammatory effects, and is suitable for widespread application.

Advantages of the third aspect of the embodiments of the present application are summarized as follows: the aforementioned pharmaceutical composition for treating pulmonary inflammatory diseases exhibits excellent anti-inflammatory effects, can effectively alleviate pulmonary inflammation, and thus can be widely applied in the preparation of drugs for chronic obstructive pulmonary disease, acute lung injury, or lower respiratory tract infections.

### DESCRIPTION OF DRAWINGS

To more clearly illustrate the technical solutions in the embodiments of this application, a brief introduction to the drawings used in the following descriptions of the embodiments or illustrative technical details is provided below. It is evident that the drawings described below represent only some embodiments of this application; a person skilled in the art may derive other drawings from these without requiring an inventive step.

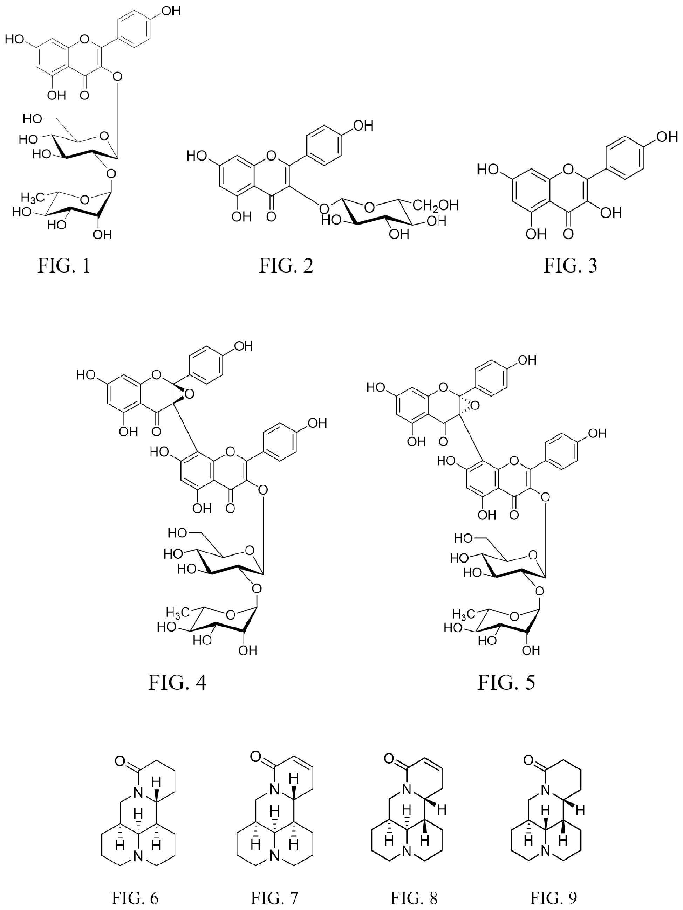

FIG. 1 shows a structural formula of kaempferol-3-O-glucorhamnoside.
FIG. 2 shows a structural formula of astragalin.
FIG. 3 shows a structural formula of kaempferol.
FIG. 4 shows a structural formula of Thesiuside A.
FIG. 5 shows a structural formula of Thesiuside B.
FIG. 6 shows a structural formula of matrine.
FIG. 7 shows a structural formula of sophocarpine.
FIG. 8 shows a structural formula of Thesiumine A.
FIG. 9 shows a structural formula of Thesiumine B.

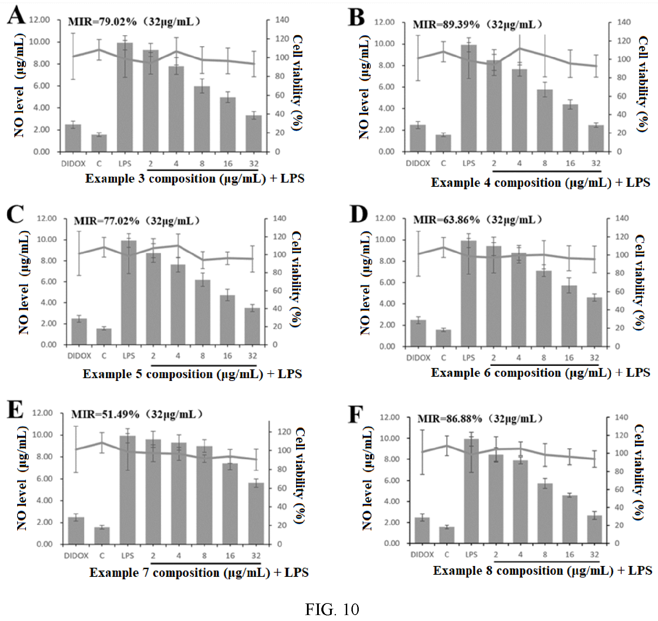

FIG. 10 shows the anti-inflammatory effects of different pharmaceutical compositions, indicating that each composition can dose-dependently inhibit the excessive production of NO in LPS-induced RAW264.7 mouse macrophages to exert anti-inflammatory effects, in which, part A of FIG. 10 shows the anti-inflammatory effect of the composition of Example 3, part B of FIG. 10 shows the anti-inflammatory effect of the composition of Example 4, part C of FIG. 10 shows the anti-inflammatory effect of the composition of Example 5, part D of FIG. 10 shows the anti-inflammatory effect of the composition of Example 6, part E of FIG. 10 shows the anti-inflammatory effect of the composition of Example 7, and part F of FIG. 10 shows the anti-inflammatory effect of the composition of Example 8.
FIG. 11 shows bar charts of the results from the NO production inhibition experiments, indicating that the Bairuisu component can dose-dependently inhibit the excessive production of NO in LPS-induced RAW264.7 mouse macrophages, in which, part A of FIG. 11 shows the bar chart of the NO production inhibition results for kaempferol, part B of FIG. 11 shows the bar chart of the NO production inhibition results for Thesiuside A, and part C of FIG. 11 shows the bar chart of the NO production inhibition results for Thesiuside B.
FIG. 12 shows bar charts of the QR-inducing activity test results, indicating that the Bairuisu component has an inhibitory effect on oxidative stress, in which, part A of FIG. 12 shows the bar chart of the QR-inducing activity test results for kaempferol, part B of FIG. 12 shows the bar chart of the QR-inducing activity test results for Thesiuside A, and part C of FIG. 12 shows the bar chart of the QR-inducing activity test results for Thesiuside B.
FIG. 13 shows the effect of the Bairuisu component Thesiuside A on the transcriptome of human bronchial epithelial Beas-2B cells.
FIG. 14 shows the effect of the matrine component Thesiumine A on the transcriptome of human bronchial epithelial Beas-2B cells.
FIG. 15 shows the potential anti-inflammatory functional genes (targets) of the Bairuisu component Thesiuside A and the matrine component Thesiumine A.

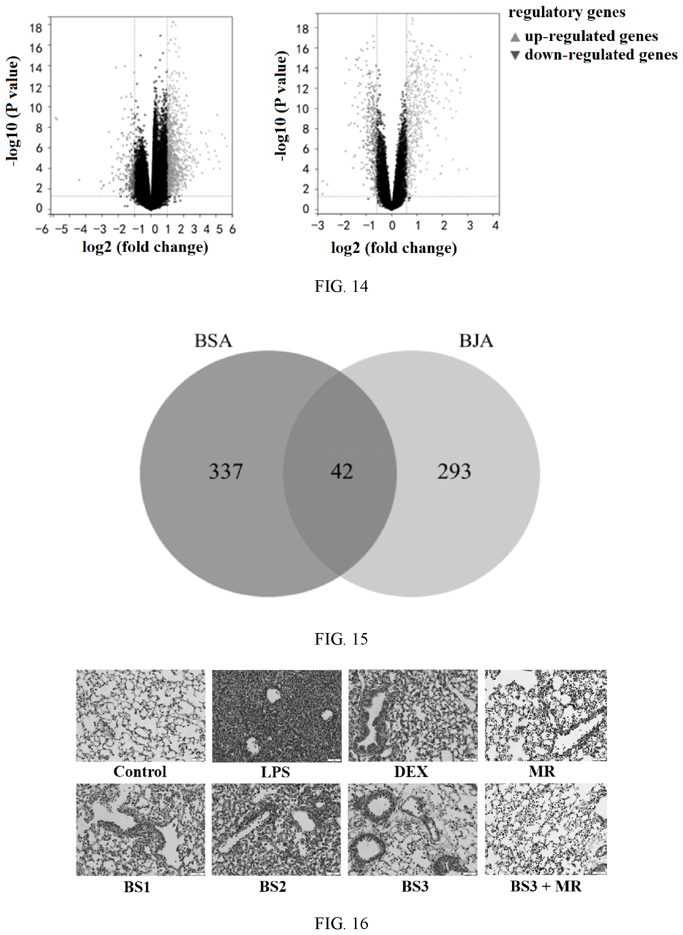

FIG. 16 shows H&E-stained images of mouse lung tissue, indicating that the Bairuisu components (kaempferol-3-O-glucorhamnoside (BS1), astragalin (BS2), and kaempferol (BS3)), the matrine component (MR), and the combination of the two classes of components (BS3 + MR) are all capable of alleviating LPS-induced pathological changes in mouse lung tissue, with the combination group showing a more pronounced effect. The concentration of the positive control drug DEX was 1 mg/kg, the LPS concentration was 10 mg/kg, and the concentrations of BS1, BS2, BS3, and MR were all 20 mg/kg; the pharmaceutical composition (BS3 + MR) consisted of 20 mg/kg of BS3 and 20 mg/kg of MR.

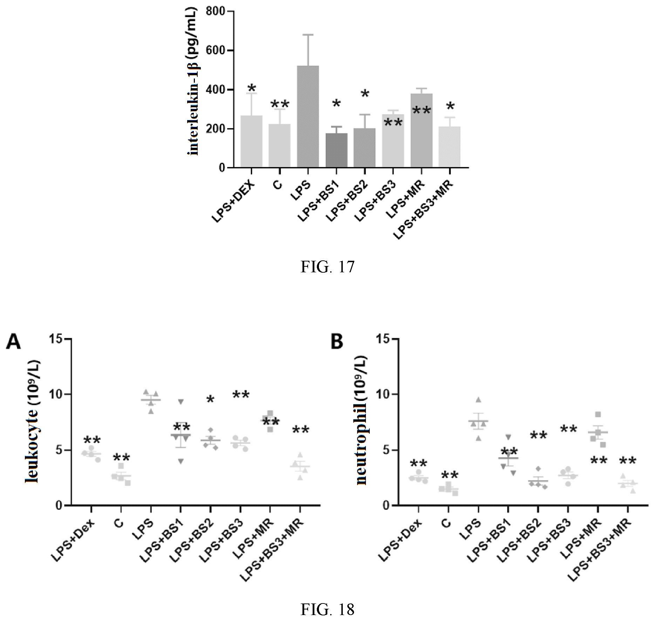

FIG. 17 shows a bar chart of the ELISA results, indicating that the Bairuisu components (kaempferol-3-O-glucorhamnoside (BS1), astragalin (BS2), and kaempferol (BS3)), the matrine component (MR), and the combination of the two classes of components (BS3 + MR) can all inhibit the LPS-induced increase of the inflammatory cytokine IL-1β in mouse bronchoalveolar lavage fluid, in which, the activity of the combination is significantly enhanced. The concentration of the positive control drug DEX was 1 mg/kg, the LPS concentration was 10 mg/kg, and the concentrations of BS1, BS2, BS3, and MR were all 20 mg/kg; the pharmaceutical composition (BS3 + MR) consisted of 20 mg/kg of BS3 and 20 mg/kg of MR.
FIG. 18 shows a scatter plot of the complete blood count results in mouse blood, indicating that the Bairuisu components (kaempferol-3-O-glucorhamnoside (BS1), astragalin (BS2), and kaempferol (BS3)), the matrine component (MR), and the combination of the two classes of components (KF + MR) can all reduce the number of inflammatory cells in mouse blood, in which, the activity of the combination is significantly enhanced. The concentration of the positive control drug DEX was 1 mg/kg, the LPS concentration was 10 mg/kg, and the concentrations of BS1, AG, BS3, and MR were all 20 mg/kg; the pharmaceutical composition (BS3 + MR) consisted of 20 mg/kg of BS3 and 20 mg/kg of MR.

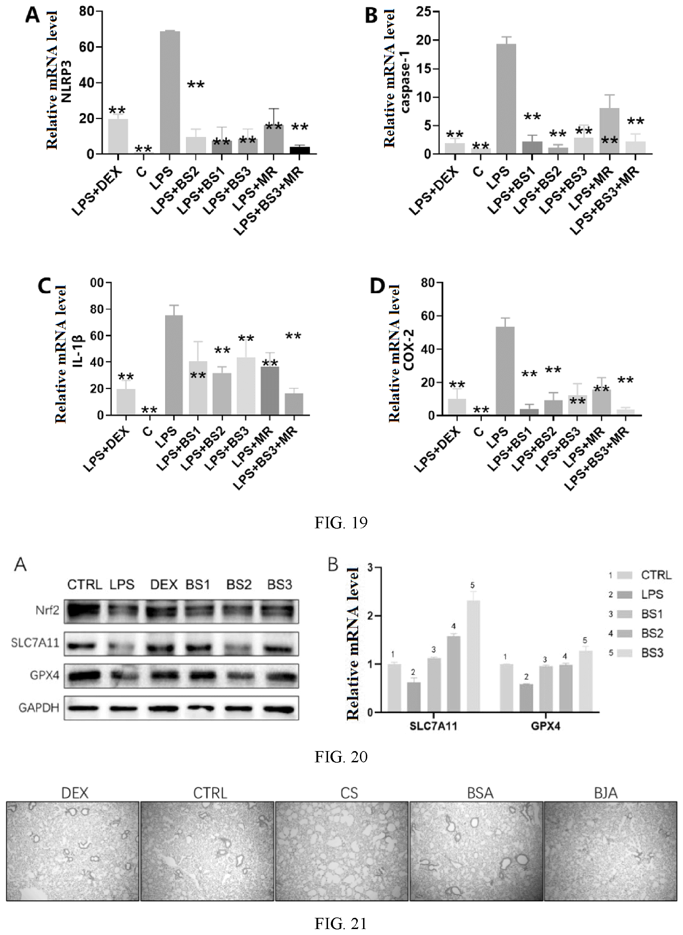

FIG. 19 shows a bar chart of the qRT-PCR results, indicating that the Bairuisu components (kaempferol-3-O-glucorhamnoside (BS1), astragalin (BS2), and kaempferol (BS3)), the matrine component (MR), and the combination of the two classes of components (BS3 + MR) can all reduce the increase in mRNA levels of inflammatory factors in mouse lung tissue, in which, the activity of the combination is significantly enhanced. The concentration of the positive control drug DEX was 1 mg/kg, the LPS concentration was 10 mg/kg, and the concentrations of BS1, BS2, BS3, and MR were all 20 mg/kg; the pharmaceutical composition (BS3 + MR) consisted of 20 mg/kg of BS3 and 20 mg/kg of MR.
FIG. 20 shows bar charts of the Western blot and qRT-PCR results, indicating that the Bairuisu component can regulate the levels of ferroptosis-related pathway proteins SLC7A11 and GPX4 as well as their mRNA levels. The concentration of the positive control drug DEX was 1 mg/kg, the LPS concentration was 10 mg/kg, and the concentrations of BS1, BS2, BS3, and MR were all 20 mg/kg.
FIG. 21 shows H&E-stained images of mouse lung tissue, indicating that both the Bairuisu component and the matrine component can attenuate CS-induced pathological damage in mouse lungs.
FIG. 22 is a bar chart of ELISA results, indicating that both the Bairuisu component and the matrine component can inhibit the production of the inflammatory cytokines IL-1β and TNF-α in CS-induced mouse lungs.
FIG. 23 is a bar chart of complete blood count results in mouse blood, indicating that both the Bairuisu component and the matrine component reduce the number of inflammatory cells in CS-induced mouse peripheral blood.
FIG. 24 is a bar chart of flow cytometry results, indicating that both the Bairuisu component and the matrine component suppress the proportion of Th1 cells in CS-induced mouse lung tissue, thereby improving the balance between Th1 and Th2 cells.
FIG. 25 is a bar chart of flow cytometry results, indicating that both the Bairuisu component and the matrine component suppress the proportion of Th17 cells in CS-induced mouse lung tissue, thereby improving the balance between Th17 and Treg cells.
FIG. 26 is a bar chart of the results regarding the improvement of the gut microbiota, indicating that the Bairuisu component improves the composition of the gut microbiota. Specifically, the Bairuisu component primarily exerts its beneficial effects on COPD by improving the Lactobacillus murinus strain, while the matrine component primarily does so through the *Bacteroides caecimuris* and *Bacteroides vulgatus* strains.
FIG. 27 is a line graph of cell viability, indicating that the combination of kaempferol (BS3) and Thesiuside A (BSA) with the matrine component (MR) can reduce the toxicity of MR to liver cells. The combination ratio of BS3 and Thesiuside A to MR is 1:1.
FIG. 28 is a line graph of cellular reactive oxygen species (ROS) levels, indicating that the combination of kaempferol (BS3) and Thesiuside A (BSA) with the matrine component (MR) can reduce the ability of MR to induce ROS in liver cells. The combination ratio of BS3 and BSA to MR is 1:1.
FIG. 29 shows the ¹H NMR spectrum of Thesiuside A.

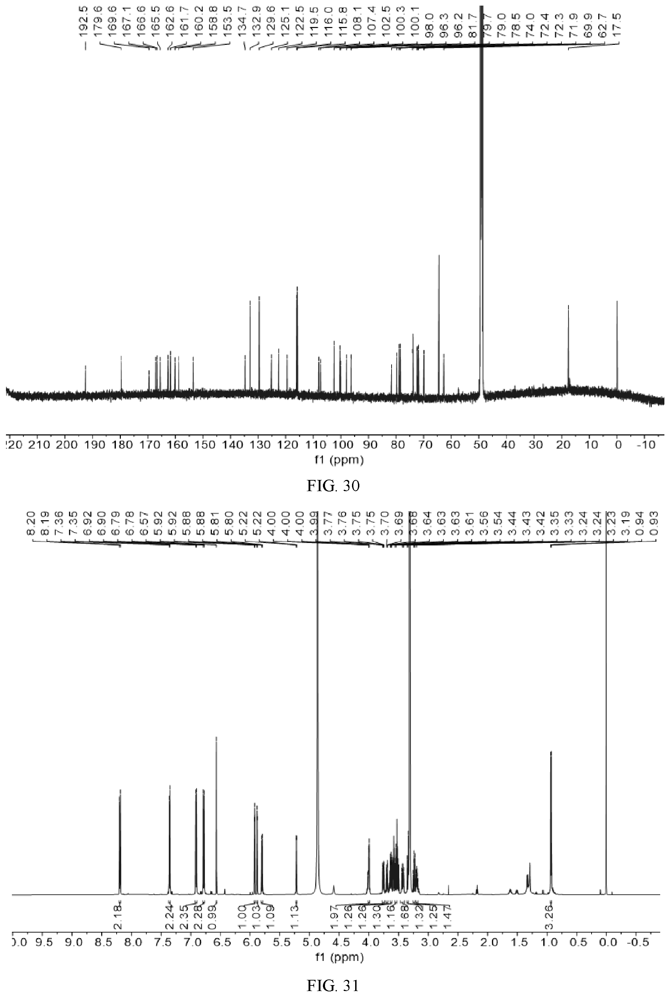

FIG. 30 shows the ¹³C NMR spectrum of Thesiuside A.
FIG. 31 shows the ¹H NMR spectrum of Thesiuside B.

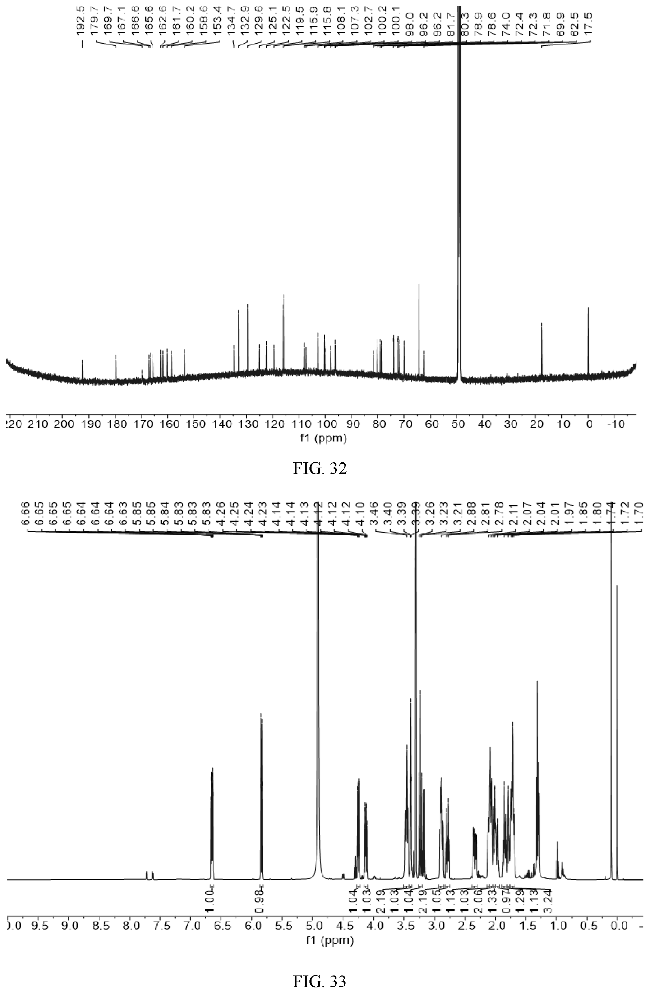

FIG. 32 shows the ¹³C NMR spectrum of Thesiuside B.
FIG. 33 shows the ¹H NMR spectrum of Thesiumine A.

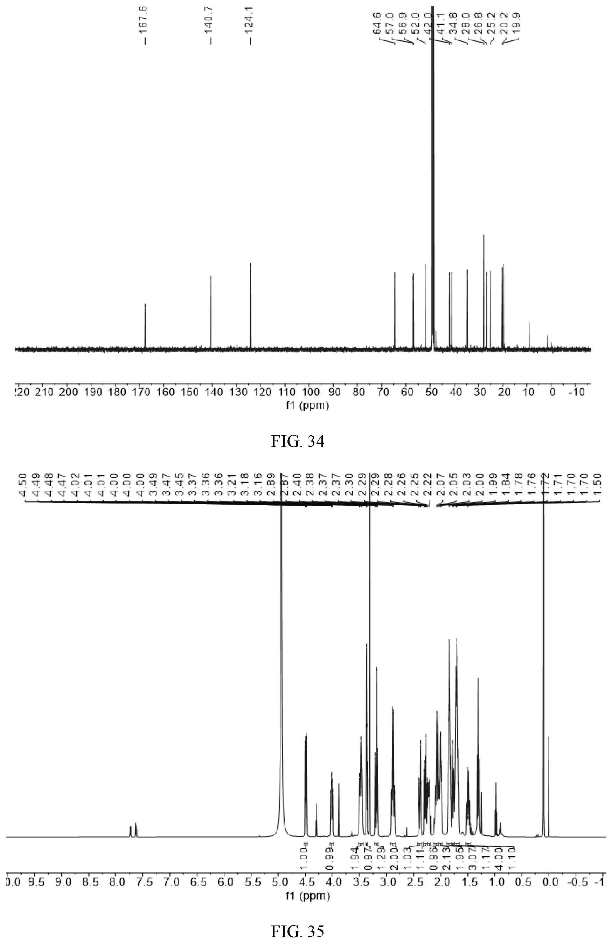

FIG. 34 shows the ¹³C NMR spectrum of Thesiumine A.
FIG. 35 shows the ¹H NMR spectrum of Thesiumine B.

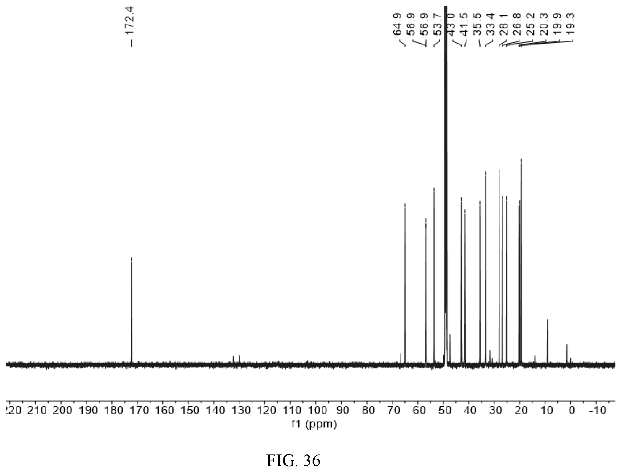

FIG. 36 shows the ¹³C NMR spectrum of Thesiumine B.

### DETAILED DESCRIPTION OF EMBODIMENTS

To make the objectives, technical solutions, and advantages of the present application clearer, the present application will be described in further detail below with reference to the accompanying drawings and embodiments. It should be understood that the specific embodiments described herein are intended to explain the present application and are not intended to limit the present application.

It should be noted that when a component is described as being "fixed to" or "mounted on" another component, it may be directly on the other component or indirectly on the other component. When a component is described as being "connected to" another component, it may be directly or indirectly connected to the other component. The terms "up," "down," "left," "right," and similar terms indicating orientation or positional relationships are based on the orientation or positional relationships shown in the drawings and are provided solely for the convenience of description, rather than to indicate or imply that the devices or elements referred to must have a specific orientation, be constructed in a specific orientation, or operate in a specific orientation. Therefore, they should not be construed as limiting the present application; a person skilled in the art may understand the specific meaning of the above terms based on the specific circumstances. The terms "first" and "second" are used solely for descriptive purposes and should not be construed as indicating or implying relative importance or the number of technical features. "Multiple" means two or more, unless otherwise explicitly specified.

To illustrate the technical solutions provided by the present application, a detailed description is provided below with reference to specific drawings and embodiments.

Embodiments of a first aspect of the present application provide a pharmaceutical composition for treating pulmonary inflammatory disease, comprising the following components:
1 to 100 parts of a Bairuisu component, and
1 to 20 parts of a matrine component.

The Bairuisu component has a structural formula as shown in Formula A: 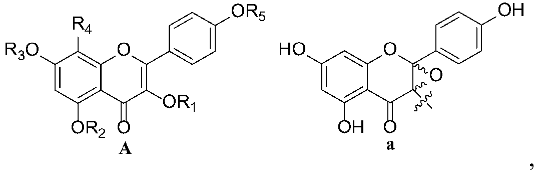 in which, R₄ represents H or group a; R₁, R₂, R₃, and R₅ are independently selected from one of the following groups: H, CH₃, CH₃CO, glucopyranosyl, rhamnopyranosyl, glucorhamnosyl, diglucosyl, xylopyranosyl, sucrosyl.

The matrine component has a structural formula shown in Formula B: 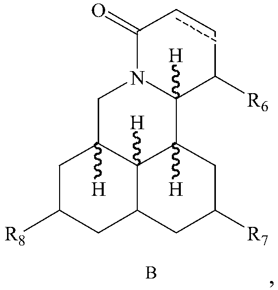 in which, R₆, R₇, and R₈ are independently selected from one of the following groups: H, OH, OCH₃, or CH₃COO.

Advantages of the embodiments of the first aspect of the present application are summarized as follows: the pharmaceutical composition provided by the present application mainly comprises a Bairuisu component and a matrine component. The Bairuisu component has QR-inducing activity, is capable of inhibiting the excessive production of NO in LPS-induced RAW264.7 mouse macrophages, alleviating LPS-induced pathological changes in mouse lung tissue, inhibiting the LPS-induced increase of the inflammatory cytokine IL-1β in mouse bronchoalveolar lavage fluid, and reducing the number of inflammatory cells in mouse blood, thereby exerting a lung-protective effect and alleviating pulmonary inflammation.

In particular, the effects of the Bairuisu component and the matrine component on the transcriptome of human bronchial epithelial Beas-2B cells were examined. Significant differences exist in the inflammatory regulatory genes modulated by the Bairuisu component and the matrine component, indicating that the anti-inflammatory mechanisms of the two types of components differ.

The Bairuisu component and the matrine component have a synergistic effect, and they exert therapeutic effects on pulmonary diseases through different mechanisms. The present application has identified a novel mechanism by which the Bairuisu component treats acute lung injury by regulating ferroptosis-related pathway proteins SLC7A11 and GPX4. Concurrently, the matrine component can inhibit the excessive activation of the NLRP3 inflammasome and suppress NF-κB activation. Furthermore, the Bairuisu component and the matrine component exert regulatory effects on different gut microbiota. Specifically, the Bairuisu component primarily increases the abundance of *Lactobacillus murinus*, while the matrine component primarily increases the abundance of *Bacteroides caecimuris* and *Bacteroides vulgatus*. It was also found that changes in these bacterial strains correlate with the expression of immune factors. The Bairuisu component and the matrine component exert synergistic lung-protective effects through different mechanisms.

The binding interactions of the Bairuisu component and the matrine component with key inflammatory regulatory proteins: the newly discovered Bairuisu components Thesiuside A and Thesiuside B exhibit significantly superior binding affinity for iNOS and COX-2 compared to the known Bairuisu component kaempferol; the newly discovered matrine component Thesiumine B also exhibits superior binding affinity for iNOS compared to the known matrine components matrine and sophocarpine. The newly discovered Bairuisu components and matrine component have better anti-inflammatory effects, and these newly discovered compounds also have good development value.

The detoxifying effect of the Bairuisu component on high-concentration matrine component: the Bairuisu component can inhibit the hepatotoxicity and nephrotoxicity of the matrine component and can suppress ROS production induced by the matrine component.

Furthermore, it is described that the matrine component can increase the water solubility of the Bairuisu component, thereby improving water solubility and bioavailability.

Moreover, the interaction between the Bairuisu component and the matrine component characterized by "synergistic efficacy, reduced toxicity, complementary mechanisms, and mutual solubilization" is described, along with the necessity and value of forming a pharmaceutical composition from these two types of components.

In some embodiments, the pharmaceutical composition comprises the following components:
60 to 66 parts of the Bairuisu component, and
2 to 5 parts of the matrine component.

In some embodiments, the Bairuisu component is selected from at least one of kaempferol-3-O-glucorhamnoside, astragalin, kaempferol, Thesiuside A, and Thesiuside B.

In some embodiments, the matrine component is selected from at least one of matrine, sophocarpine, Thesiumine A, and Thesiumine B.

In some embodiments, the pharmaceutical composition comprises the following components:
10 to 20 parts of Thesiuside A,
10 to 20 parts of Thesiuside B,
1 to 2 parts of Thesiumine A, and
1 to 2 parts of Thesiumine B.

In some embodiments, the pharmaceutical composition comprises the following components:
10 to 15 parts of kaempferol, and
1 part of matrine.

In some specific embodiments, the pharmaceutical composition comprises the following components:
12 parts of kaempferol, and
1 part of matrine.

In some embodiments, an extraction method of the Thesiuside A and the Thesiuside B comprises the following steps:
crushing whole herbs of dried *Thesium chinense* Turcz., soaking at 20°C to 40°C for 1 hr to 10 hrs, followed by extraction by heating under reflux with 60% to 80% ethanol to obtain an extract solution; filtering and concentrating the extract solution in sequence to obtain a total crude extract;
suspending the total crude extract in water, then sequentially extracting with petroleum ether, dichloromethane and ethyl acetate to obtain organic extract solutions;
subjecting an ethyl acetate extract solution to a normal-phase silica gel column chromatography, in which, a gradient elution is performed first with a petroleum ether/ethyl acetate eluent in a volume ratio of (0.1 to 1) : (0.1 to 1), and then with an ethyl acetate/methanol eluent in a volume ratio of (0.1 to 1) : (0.1 to 1); collecting eight fractions in chronological order to obtain first elution fractions E1 to E8;
subjecting a first elution fraction E7 to an MCI column to remove pigments, performing gradient elution with a methanol/water eluent in a volume ratio of (1 to 3) : ( 0.1 to 7), collecting eight fractions in chronological order to obtain second elution fractions E7a to E7h;
subjecting a second elution fraction E7d to normal-phase silica gel column chromatography, performing gradient elution with a dichloromethane/methanol eluent in a volume ratio of (0.1 to 30) : 1, collecting eight fractions in chronological order to obtain third elution fractions E7a to E7h; and
subjecting a third elution fraction E7d5 to separation and purification using Sephadex LH-20 and C18 reverse-phase silica gel to obtain the compounds Thesiuside A (purity >98%) and Thesiuside B (purity >98%).

Thesiuside A has a structural formula as follows: 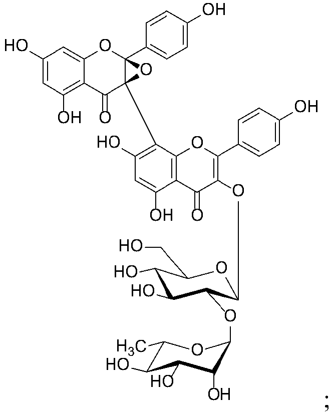 and
Thesiuside B has a structural formula as follows:

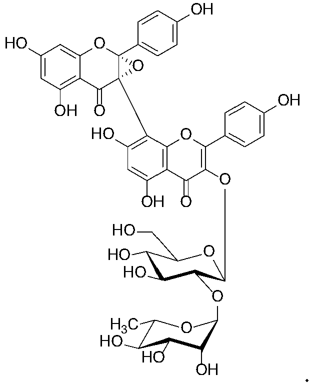

In this process, the total crude extract is obtained by concentrating the ethanol extract. To enable the subsequent extraction process to better separate the extract into several fractions with different polarities, the ethanol must be almost completely recovered during concentration. At this point, the total crude extract becomes very viscous and cannot be extracted directly; therefore, it is necessary to disperse it using water (a solvent with high polarity), followed by sequential extraction with solvents of increasing polarity.

In some specific embodiments, when using Sephadex LH-20 for separation, the elution program employing methanol/water gradient elution is as follows: 3:7→4:6.

In some embodiments, an extraction method of Thesiumine A and Thesiumine B comprises the following steps:
crushing whole herbs of dried *Thesium chinense* Turcz., extracting with an aqueous hydrochloric acid solution having a mass concentration of 0.1 mol/L to 0.5 mol/L (after acid-water extraction, the alkaloids exist in the acidic solution as salts; adding a base precipitates the alkaloids, thereby facilitating thorough extraction with dichloromethane), adjusting a resulting extract solution to an alkaline pH, repeatedly extracting with dichloromethane, and concentrating under reduced pressure to obtain a total alkaloid extract;
subjecting the total alkaloid extract to a normal-phase silica gel column chromatography, eluting with a petroleum ether/dichloromethane/ammonia water eluent in a volume ratio of 1: 1: (0.02 to 0.03); performing gradient elution with a dichloromethane/methanol eluent in a volume ratio of (6 to 97): (3 to 4), collecting 10 fractions in chronological order to obtain fourth elution fractions S1 to S10;
purifying a fourth elution fraction S5 by Sephadex LH-20 column chromatography using methanol as an eluent, subjecting a purified S5 fraction to HPLC separation using a mobile phase having a pH of 3 to 4 and a methanol volume percentage of 5% to 10%; and
removing impurities through a macroporous adsorption resin column to obtain Thesiumine B and Thesiumine A.

Thesiumine A has a structural formula as follows: 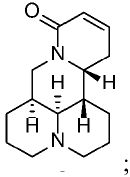 and
Thesiumine B has a structural formula as follows:

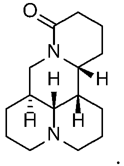

In some embodiments, the extraction process for kaempferol-3-O-glucorhamnoside, astragalin, and kaempferol comprises the following steps:
crushing whole herbs of dried *Thesium chinense* Turcz., soaking at 20°C to 40°C for 1 hr to 10 hrs, followed by extraction by heating under reflux with an aqueous ethanol solution having a mass percentage of 60% to 80% to obtain an extract solution; filtering and concentrating the extract solution in sequence to obtain a total crude extract;
suspending the total crude extract in water, then sequentially extracting with petroleum ether, dichloromethane and ethyl acetate to obtain organic extract solutions;
diluting an ethyl acetate extract solution with water, passing a resulting mixture through an MCI column, and then eluting sequentially with aqueous methanol solutions having mass percentages of 20 to 30%, 40 to 50%, and 60 to 70%, yielding a first fraction, a second fraction, and a third fraction, respectively;
further eluting the first fraction sequentially with pure water, aqueous ethanol solutions having mass percentages of 20% to 30%, 40% to 50%, and 60% to 70%, and pure ethanol, yielding a fraction a, a fraction b, a fraction c, a fraction d, and a fraction e, respectively;
purifying the fraction b by gel column chromatography using methanol as an eluent, followed by recrystallization to obtain kaempferol-3-O-glucorhamnoside (purity >98%);
purifying the fraction c by gel column chromatography using methanol as the eluent, followed by recrystallization to obtain astragalin (purity >98%); and
purifying the third fraction by gel column chromatography using methanol as the eluent, and followed by recrystallization to obtain kaempferol (purity >98%).

The MCI column is an MCI gel column, which is a microporous resin gel column (with polystyrene-based reversed-phase resin packing). The macroporous resin column is an AB-8 type macroporous resin column; the gel chromatography column is a Sephadex LH-20 gel chromatography column.

Embodiments of a second aspect of the present application provide a preparation for treating pulmonary inflammatory diseases, comprising: the aforementioned pharmaceutical composition as the active ingredient, having a mass percentage in the preparation of 0.1% to 99.9%, with a remainder being pharmaceutically acceptable excipients. A dosage form of the preparation is a tablet, sugar-coated tablet, film-coated tablet, enteric-coated tablet, capsule, oral solution, buccal tablet, granules, oral granules, pill, powder, ointment, pellet, suspension, fine powder, injection, suppository, cream, spray, drop, or patch.

Advantages of the embodiments of the second aspect of the present application are summarized as follows: in the provided preparation for treating pulmonary inflammatory diseases, the aforementioned pharmaceutical composition constitutes 0.1% to 99.9% by mass of the composition as an active ingredient; thus, the provided preparation alleviates pulmonary inflammation, protects the lungs, exhibits excellent anti-inflammatory effects, and is suitable for widespread application.

The composition of the present application is formulated into a unit-dose pharmaceutical dosage form, in which, the term "unit-dose form" refers to a single unit of the preparation, such as each tablet, each capsule, each bottle of oral solution, or each sachet of granules.

Preferably, the dosage forms of the preparation are capsules, tablets, oral solutions, granules, pills, powders, pellets, or ointments.

The oral dosage forms may contain commonly used excipients, binders, fillers, diluents, tableting agents, lubricants, disintegrants, colorants, flavorings, and wetting agents; tablets may be coated if necessary.

Suitable excipients include lactose, D-mannitol, D-sorbitol, starches such as α-starch, dextrin, microcrystalline cellulose, low-substituted hydroxypropyl cellulose, sodium carboxymethyl cellulose, gum arabic, amylopectin, light anhydrous silicic acid, synthetic aluminum silicate, and magnesium aluminum silicate. Suitable binders include: α-starch, sucrose, gelatin, gum arabic, methylcellulose, carboxymethyl cellulose, sodium carboxymethyl cellulose, crystalline cellulose, sugars, D-mannitol, trehalose, dextrin, amylopectin, hydroxypropyl cellulose, hydroxypropyl methylcellulose, pyrrolidone, etc. Suitable fillers include cellulose, mannitol, lactose, and other similar fillers. Suitable disintegrants include lactose, sugar, starch, carboxymethyl cellulose, calcium carboxymethyl cellulose, sodium alkyl sulfate, sodium carboxymethyl starch, light anhydrous silicic acid, polyvinylpyrrolidone, and sodium starch glycolate, etc. Suitable lubricants include magnesium stearate, calcium stearate, talc, silica gel, and sodium lauryl sulfate. Suitable pharmaceutically acceptable wetting agents include sodium lauryl sulfate. Suitable coating agents include hydroxypropyl methylcellulose, hydroxypropyl cellulose, ethyl cellulose, carboxymethyl cellulose, polyvinyl alcohol, etc. Suitable colorants include water-soluble food-grade citrazine dyes (e.g., Food Red No. 2 and No. 3, Food Yellow No. 4 and No. 5, Food Blue No. 1 and No. 2); water-insoluble lakes (e.g., aluminum salts of the aforementioned water-soluble food citrazine dyes); and natural dyes (e.g., β-carotene, chlorophyll, iron oxide red), etc. Suitable sweeteners include: sodium saccharin, glycyrrhetinic acid, aspartame, stevia, etc.

For injectable preparations, the prepared liquid unit dosage form contains the active substance of the present application and a sterile carrier. Depending on the carrier and concentration, the compound may be suspended or dissolved. The solution is typically prepared by dissolving the active substance in a carrier, followed by filter sterilization before filling into suitable vials or ampoules, and then sealing. Excipients such as a local anesthetic, preservatives, and buffering agents may also be dissolved in this carrier. To enhance stability, the composition may be frozen after filling into vials, and water may be removed under vacuum.

The composition of the present application may optionally incorporate suitable pharmaceutically acceptable carriers when being formulated into a medicament. The pharmaceutically acceptable carriers are selected from: mannitol, sorbitol, sodium metabisulfite, sodium bisulfite, sodium thiosulfate, cysteine hydrochloride, thioglycolic acid, methionine, vitamin C, disodium EDTA, sodium calcium EDTA, carbonates, acetates, or phosphates of monovalent alkali metals or aqueous solutions thereof, hydrochloric acid, acetic acid, sulfuric acid, phosphoric acid, amino acids, sodium chloride, potassium chloride, sodium lactate, xylitol, maltose, glucose, fructose, dextrose, glycine, starch, sucrose, lactose, mannitol, silicon derivatives, cellulose and its derivatives, alginates, gelatin, polyvinylpyrrolidone, glycerin, Tween 80, agar, calcium carbonate, calcium bicarbonate, surfactants, polyethylene glycol, cyclodextrin, beta-cyclodextrin, phospholipid materials, kaolin, talc, calcium stearate, magnesium stearate, etc.

Embodiments of the third aspect of the present application provide an application of the aforementioned pharmaceutical composition for treating the pulmonary inflammatory diseases in the manufacturing of a medicament for treating chronic obstructive pulmonary disease, acute lung injury, or lower respiratory tract infections.

Advantages of the embodiments of the third aspect of the present application are summarized as follows: the aforementioned pharmaceutical composition for treating pulmonary inflammatory diseases exhibits excellent anti-inflammatory effects, can effectively alleviate pulmonary inflammation, and thus can be widely applied in the preparation of drugs for chronic obstructive pulmonary disease, acute lung injury, or lower respiratory tract infections.

Specific examples are provided below for illustrative purposes.

The compounds kaempferol-3-O-glucorhamnoside, astragalin, and kaempferol described in this application are known Bairuisu components, and their structures were determined using ¹H NMR and ¹³C NMR spectra in conjunction with a literature comparison.

This application is further described below with reference to the examples.

### Example 1

This example describes the preparation methods for five representative Bairuisu components and two representative matrine components. The details are as follows:

Kaempferol-3-O-glucorhamnoside, astragalin, and kaempferol provided in the present application were prepared by the following methods:

### (1) Preparation of Thesium chinense Turcz. extract

Whole herbs of dried *Thesium chinense* Turcz. were crushed, soaked at room temperature for 3 hours, and then extracted three times with 75% ethanol (each extraction lasting 3 hours). The extract was filtered and concentrated under vacuum in a rotary evaporator to obtain a total crude extract.

### (2) Extraction

The total crude extract was suspended in water and extracted four times sequentially with equal volumes of petroleum ether, dichloromethane, and ethyl acetate to obtain respective organic layers.

### (3) Impurity removal

The ethyl acetate layer was dissolved in water and passed through an MCI column. Elution was performed with 30%, 50%, and 70% aqueous methanol solutions to remove pigments and some impurities. The 30% methanol-water fraction (A) and the 70% methanol-water fraction (B) were used for further separation.

### (4) Purification

Fraction A was subjected to column chromatography on an AB-8 macroporous resin column. Gradient elution was performed sequentially with pure water, 30% ethanol-water, 50% ethanol-water, 70% ethanol-water, and pure ethanol, each for three column volumes. The 30% ethanol-water fraction (C) and the 50% ethanol-water fraction (D) were used for further purification. Fraction C was purified using a Sephadex LH-20 gel chromatography column with methanol as the eluent, and then recrystallized using methanol as a solvent at 4°C to obtain high-purity kaempferol-3-O-glucorhamnoside with a purity of 99% and a yield of 82%.

Fraction D was purified on a Sephadex LH-20 gel column using methanol as the eluent, and then recrystallized to obtain high-purity astragalin with a purity of 99% and a yield of 78%.

Fraction B was purified on a Sephadex LH-20 gel column using methanol as the eluent, and then recrystallized to obtain high-purity kaempferol with a purity of 99% and a yield of 85%.

Thesiuside A and Thesiuside B from *Thesium chinense* Turcz. as provided in the present application were prepared by the following method:

### (1) Preparation of the extract of Thesium chinense Turcz.

Whole herbs of dried *Thesium chinense* Turcz. were crushed, soaked at room temperature for 3 hours, and then extracted three times by heating under reflux with 75% ethanol for 3 hours each time. The extract was filtered and concentrated under vacuum using a rotary evaporator to obtain a total crude extract.

### (2) Extraction

The total crude extract was suspended in water at a volume ratio of 1:5 (total crude extract : water). The suspension was extracted four times sequentially with petroleum ether, dichloromethane, and ethyl acetate, each solvent being used in a volume six times that of the total crude extract, to obtain the respective organic layers.

### (3) Purification

The ethyl acetate fraction was first separated by normal-phase silica gel column chromatography using eluents of petroleum ether/ethyl acetate (1:0 → 0:1) and ethyl acetate/methanol (1:0 → 0:1), yielding 8 fractions (E1-E8). Fraction E7 was then subjected to MCI column chromatography to remove some pigments, using methanol/water (3:7 → 1:0) as the eluent, yielding 8 subfractions (E7a-E7h). Fraction E7d was separated by normal-phase silica gel column chromatography (dichloromethane/methanol, 30:1 → 0:1) to yield 6 subfractions (E7d1-E7d6). Fraction E7d5 was further separated and purified using Sephadex LH-20 gel and C18 reverse-phase silica gel, yielding the compounds Thesiuside A (purity 99%, yield 68%) and Thesiuside B (purity 99%, yield 71%).

The present application provides a method for preparing Thesiumine A and Thesiumine B from *Thesium chinense* Turcz., with the specific steps as follows:

### (1) Preparation of total alkaloids from Thesium chinense Turcz.

A batch of dried and crushed whole plant material of *Thesium chinense* Turcz. was taken. It was extracted with a 0.3 mol/L aqueous hydrochloric acid solution. The pH of the extract solution was adjusted to 10 using a 1.32 mol/L aqueous sodium hydroxide solution, followed by extraction with dichloromethane four times. The resulting solution was concentrated under reduced pressure to obtain a total alkaloid extract.

### (2) Purification

The total alkaloid extract was separated by normal-phase silica gel column chromatography using eluents of petroleum ether/dichloromethane/ammonia water (1:1:0.03) and dichloromethane/methanol (97:3 → 6:4), yielding a total of 10 fractions (S1-S10). Fractions S5-S7 were selected for further systematic separation. Fraction S5 was separated by C18 reverse-phase silica gel column chromatography (methanol/water, 0:1 → 1:0), yielding a total of 6 subfractions (S5a-S5f). Subfraction S5f was first purified by Sephadex LH-20 column chromatography using pure methanol as the eluent. The purified samples were further separated by C18 reverse-phase silica gel, yielding the compounds Thesiumine A (99% purity, 65% yield) and Thesiumine B (99% purity, 70% yield).

### Comparative Example 1

Kaempferol-3-O-glucorhamnoside, astragalin, and kaempferol were prepared by the following methods:

### (1) Preparation of Thesium chinense Turcz. extract

Whole herbs of dried *Thesium chinense* Turcz. were crushed, soaked at room temperature for 3 hours, and then extracted three times with 75% ethanol (each extraction lasting 3 hours). The extract was filtered and concentrated under vacuum in a rotary evaporator to obtain a total crude extract.

### (2) Extraction

The total crude extract was suspended in water and extracted four times sequentially with equal volumes of petroleum ether, dichloromethane, and ethyl acetate to obtain respective organic layers.

### (3) Impurity removal

The ethyl acetate layer was dissolved in water and passed through an MCI column. Elution was performed with 30%, 50%, and 70% aqueous methanol solutions to remove pigments and some impurities. The 30% methanol-water fraction (A) and the 70% methanol-water fraction (B) were used for further separation.

### (4) Purification

Fraction A was subjected to column chromatography on a D101 macroporous resin column. Gradient elution was performed sequentially with pure water, 30% ethanol-water, 50% ethanol-water, 70% ethanol-water, and pure ethanol, each for three column volumes. The 30% ethanol-water fraction (C) and the 50% ethanol-water fraction (D) were used for further purification. Fraction C was purified using a Sephadex LH-20 gel chromatography column with methanol as the eluent, and then recrystallized using methanol as a solvent at 4°C to obtain high-purity kaempferol-3-O-glucorhamnoside with a purity of 99% and a yield of 72%.

Fraction D was purified on a Sephadex LH-20 gel column using methanol as the eluent, and then recrystallized to obtain high-purity astragalin with a purity of 99% and a yield of 76%.

Fraction B was purified on a Sephadex LH-20 gel column using methanol as the eluent, and then recrystallized to obtain high-purity kaempferol with a purity of 99% and a yield of 83%.

### Comparative Example 2

Kaempferol-3-O-glucorhamnoside, astragalin, and kaempferol were prepared by the following methods:

### (1) Preparation of Thesium chinense Turcz. extract

Whole herbs of dried *Thesium chinense* Turcz. were crushed, soaked at room temperature for 3 hours, and then extracted three times with water (each extraction lasting 3 hours). The extract was filtered and concentrated under vacuum in a rotary evaporator to obtain a total crude extract.

### (2) Extraction

The total crude extract was suspended in water and extracted four times sequentially with equal volumes of petroleum ether, dichloromethane, and ethyl acetate to obtain respective organic layers.

### (3) Impurity removal

The ethyl acetate layer was dissolved in water and passed through an MCI column. Elution was performed with 30%, 50%, and 70% aqueous methanol solutions to remove pigments and some impurities. The 30% methanol-water fraction (A) and the 70% methanol-water fraction (B) were used for further separation.

### (4) Purification

Fraction A was subjected to column chromatography on an AB-8 macroporous resin column. Gradient elution was performed sequentially with pure water, 30% ethanol-water, 50% ethanol-water, 70% ethanol-water, and pure ethanol, each for three column volumes. The 30% ethanol-water fraction (C) and the 50% ethanol-water fraction (D) were used for further purification. Fraction C was purified using a Sephadex LH-20 gel chromatography column with methanol as the eluent, and then recrystallized using methanol as a solvent at 4°C to obtain high-purity kaempferol-3-O-glucorhamnoside with a purity of 99% and a yield of 64%.

Fraction D was purified on a Sephadex LH-20 gel column using methanol as the eluent, and then recrystallized to obtain high-purity astragalin with a purity of 99% and a yield of 62%.

Fraction B was purified on a Sephadex LH-20 gel column using methanol as the eluent, and then recrystallized to obtain high-purity kaempferol with a purity of 98% and a yield of 51%.

### Comparative Example 3

Kaempferol-3-O-glucorhamnoside, astragalin, and kaempferol were prepared by the following methods:

### (1) Preparation of Thesium chinense Turcz. extract

Whole herbs of dried *Thesium chinense* Turcz. were crushed, soaked at room temperature for 3 hours, and then extracted three times with 75% ethanol (each extraction lasting 3 hours). The extract was filtered and concentrated under vacuum in a rotary evaporator to obtain a total crude extract.

### (2) Extraction

The total crude extract was suspended in water and extracted four times sequentially with equal volumes of petroleum ether, dichloromethane, and ethyl acetate to obtain respective organic layers.

### (3) Impurity removal

The ethyl acetate layer was dissolved in water and passed through an MCI column. Elution was performed with 30%, 50%, and 70% aqueous methanol solutions to remove pigments and some impurities. The 30% methanol-water fraction (A) and the 70% methanol-water fraction (B) were used for further separation.

### (4) Purification

Fraction A was subjected to column chromatography on an AB-8 macroporous resin column. Gradient elution was performed sequentially with pure water, 30% ethanol-water, 50% ethanol-water, 70% ethanol-water, and pure ethanol, each for three column volumes. The 30% ethanol-water fraction (C) and the 50% ethanol-water fraction (D) were used for further purification. Fraction C was purified using a Sephadex LH-20 gel chromatography column with methanol as the eluent to obtain kaempferol-3-O-glucorhamnoside with a purity of 83% and a yield of 89%.

Fraction D was purified on a Sephadex LH-20 gel column using methanol as the eluent to obtain astragalin with a purity of 76% and a yield of 85%.

Fraction B was purified on a Sephadex LH-20 gel column using methanol as the eluent to obtain kaempferol with a purity of 68% and a yield of 90%.

### Comparative Example 4

Thesiuside A and Thesiuside B were prepared by the following methods:

### (1) Preparation of Thesium chinense Turcz. extract

Whole herbs of dried *Thesium chinense* Turcz. were crushed, soaked at room temperature for 3 hours, and then extracted three times with 75% ethanol by heating under reflux (each extraction lasting 3 hours). The extract was filtered and concentrated under vacuum in a rotary evaporator to obtain a total crude extract.

### (2) Extraction

The total crude extract was suspended in water at a volume ratio of the total crude extract to water of 1:5, and then extracted four times sequentially with six volumes (relative to the total crude extract) of petroleum ether, dichloromethane, and ethyl acetate, respectively, to obtain respective organic layers.

### (3) Purification

The ethyl acetate fraction was first subjected to normal-phase silica gel column chromatography using eluents of petroleum ether/ethyl acetate (1:0 → 0:1) and ethyl acetate/methanol (1:0 → 0:1), yielding 8 fractions (E1-E8). Fraction E7 was passed through an MCI column chromatography to remove some pigments using an eluent of methanol/water (3:7 → 1:0), yielding 8 subfractions (E7a-E7h). Subfraction E7d was separated by normal-phase silica gel column chromatography (dichloromethane/methanol, 30:1 → 0:1) to obtain 6 fractions (E7d1-E7d6). Subfraction E7d5 was separated and purified by Sephadex LH-20 gel chromatography to obtain the compounds Thesiuside A (purity 67%, yield 88%) and Thesiuside B (purity 71%, yield 83%).

### Comparative Example 5

Thesiuside A and Thesiuside B were prepared by the following methods:

### (1) Preparation of Thesium chinense Turcz. extract

Whole herbs of dried *Thesium chinense* Turcz. were crushed, soaked at room temperature for 3 hours, and then extracted three times with water by heating under reflux (each extraction lasting 3 hours). The extract was filtered and concentrated under vacuum in a rotary evaporator to obtain a total crude extract.

### (2) Extraction

The total crude extract was suspended in water at a volume ratio of the total crude extract to water of 1:5, and then extracted four times sequentially with six volumes (relative to the total crude extract) of petroleum ether, dichloromethane, and ethyl acetate, respectively, to obtain respective organic layers.

### (3) Purification

The ethyl acetate fraction was first subjected to normal-phase silica gel column chromatography using eluents of petroleum ether/ethyl acetate (1:0 → 0:1) and ethyl acetate/methanol (1:0 → 0:1), yielding 8 fractions (E1-E8). Fraction E7 was passed through an MCI column chromatography to remove some pigments using an eluent of methanol/water (3:7 → 1:0), yielding 8 subfractions (E7a-E7h). Subfraction E7d was separated by normal-phase silica gel column chromatography (dichloromethane/methanol, 30:1 → 0:1) to obtain 6 fractions (E7d1-E7d6). Subfraction E7d5 was separated and purified by Sephadex LH-20 gel chromatography to obtain the compounds Thesiuside A (purity 65%, yield 46%) and Thesiuside B (purity 58%, yield 41%).

### Comparative Example 6

Thesiumine A and Thesiumine B were prepared by the following methods:

### (1) Preparation of total alkaloids of Thesium chinense Turcz.

A batch of dried and crushed whole herbs of *Thesium chinense* Turcz. was extracted with a 0.3 mol/L aqueous hydrochloric acid solution. The extract solution was adjusted to pH 10 with a 1.32 mol/L aqueous sodium hydroxide solution, then extracted four times with dichloromethane. The resulting solution was concentrated under reduced pressure to obtain a total alkaloid extract.

### (2) Purification

The total alkaloid extract was separated by normal-phase silica gel column chromatography using eluents of petroleum ether/dichloromethane/ammonia water (1:1:0.03) and dichloromethane/methanol (97:3 → 6:4), yielding a total of 10 fractions (S1-S10). Fractions S5-S7 were selected for further systematic separation. Fraction S5 was separated by C18 reverse-phase silica gel column chromatography (methanol/water, 0:1 → 1:0), yielding a total of 6 subfractions (S5a-S5f). Subfraction S5f was purified by Sephadex LH-20 column chromatography using pure methanol as the eluent to obtain the compounds Thesiumine A (purity 67%, yield 79%) and Thesiumine B (purity 72%, yield 76%).

### Example 2

Structural characterization data for the compounds prepared in Example 1:
This example presented structural characterization data for five representative Bairuisu components and two representative matrine components, including the structural identification of four new compounds, as detailed below:

### (1) Structural identification of Thesiuside A

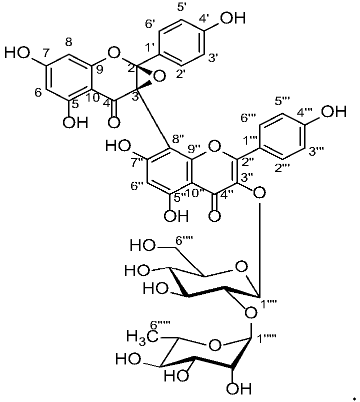

This compound was obtained as a yellow oil, soluble in methanol. Using dichloromethane/methanol as the developing solvent in a 4:1 ratio, with a drop of glacial acetic acid added, thin-layer chromatography was performed. Under a 254 nm UV lamp, a dark spot was observed. After developing the spot with a solution of p-anisaldehyde and concentrated sulfuric acid followed by heating, a yellow spot was observed, suggesting a flavonoid compound; the R*_{f}* value was 0.38. HRESIMS showed a [M+H]⁺ peak at *m*/*z* 879.1991 (calculated value: 879.1978), leading to the molecular formula C₄₂H₃₈O₂₁.

¹H NMR (600 MHz, CD₃OD) spectrum revealed two sets of AA'BB' spin coupling systems: ring B [*δ*_{H} 7.35 (2H, d, *J* = 8.9 Hz) and 6.78 (2H, d, *J* = 8.9 Hz)] and ring E [*δ*_{H} 8.12 (2H, d, *J* = 9.0 Hz) and 6.90 (2 H, d, *J* = 9.0 Hz)]. In addition, there was a tri-substituted aromatic ring, ring A [*δ*_{H} 5.96 (¹H, d, *J* = 2.1 Hz) and 5.90 (¹H, d, *J* = 2.1 Hz)], and an aromatic proton signal on ring D, *δ*_{H} 6.57 (¹H, s). The presence of two sugar terminal proton signals at *δ*_{H} 5.74 (¹H, d, *J* = 7.7 Hz) and 5.23 (¹H, d, *J =* 1.9 Hz) indicated the presence of two sugars. Furthermore, the presence of multiple overlapping peaks in the 3-4 chemical shift range further confirmed the presence of two sugars. The ¹³C NMR (150 MHz, CD₃OD) spectrum shows 38 carbon signals; combined with the HSQC spectrum, two ketone carbonyl signals (*δ*_{C} 192.5, 179.6), two double bond carbon signals (*δ*_{C} 158.8, 134.7), and twenty-four aromatic carbon signals were identified. Furthermore, the carbon signals at *δ*_{C} 119.5 and *δ*_{C} 81.7 were found to be two oxygen-bonded carbon signals; based on the number of oxygen atoms in the molecular formula, it was concluded that these signals correspond to an epoxane structure formed by the carbon atoms at positions C-2 and C-3.

Furthermore, based on the relationships between H-2' and C-2, as well as H-2‴ and C-2", it was determined that Ring B is connected to Ring C and Ring D is connected to Ring E. Consequently, the compound was identified as a diflavonoglycoside composed of a kaempferol-type moiety (rings A, B, and C), an epoxyflavanone-type moiety, and two sugar substituents. C-3 and C-8" represented the only possible linkage between the two flavanone units. Further, based on HSQC, ¹H-¹H COSY, and HMBC spectra, two glycosidic signals were assigned: β-glucose [*δ*_{H} 5.74 (¹H, d, *J* = 7.7 Hz), 3.73 (¹H, d, *J =* 12.0, 2.1 Hz), 3.63 (¹H, dd, *J =* 9.2, 7.7 Hz), 3.55 (¹H, dd, *J =* 9.2, 8.7 Hz), 3.49 (¹H, dd, *J* = 12.0, 5.9 Hz), 3.27 (¹H, dd, *J* = 9.8, 8.7 Hz), and 3.23 (¹H, ddd, *J* = 9.8, 5.9, 2.1 Hz), as well as *δ*_{C} 100.1, 79.7, 79.0, 71.9, 78.5, and 62.9] and an α-rhamnopyranosyl unit [*δ*_{H} 5.23 (¹H, d, *J* = 1.9 Hz), 3.98 (¹H, dd, *J =* 3.4, 1.9 Hz), 3.98 (¹H, overlap), 3.73 (¹H, dd, *J* = 7.8, 1.7 Hz), 3.32 (¹H, overlap), and 0.91 (3H, d, *J* = 6.2 Hz) and *δ*_{C} 102.5, 72.4, 72.3, 74.0, 69.9, and 17.5]. The correlation between H-1ʺʺ/C-3" and H-1‴ʺ/C-2ʺʺ in the HMBC spectrum established the linkage between the two sugar moieties. The relative configuration of the compound was thus deduced, and its structure was found to be similar to that of balsamiside A reported in the literature, with the main difference being the linkage positions of the two sugar units.

C-2 and C-3 are two chiral carbons; their absolute configurations were determined by comparing the ECD spectrum of this compound with those reported in the literature. The ECD spectrum shows a positive Cotton effect at 320 nm and a negative Cotton effect at 288 nm. The diflavone structure of this compound was identical to that of the hydrolysis product of balsamiside A reported in the literature, and its Cotton effects were also essentially consistent, thereby confirming its absolute configuration as 2S,3S. In summary, the structure of this compound was [(2S,3S)-2,3-epoxy-5,7,4'- trihydroxyflavanone]-(3→8)-kaempferol 3"-*O*-*α*-L-rhamnopyranosyl-(1→2)-*β*-D- glucopyranoside, and it is named Thesiuside A.

### (2) Structural characterization of Thesiuside B

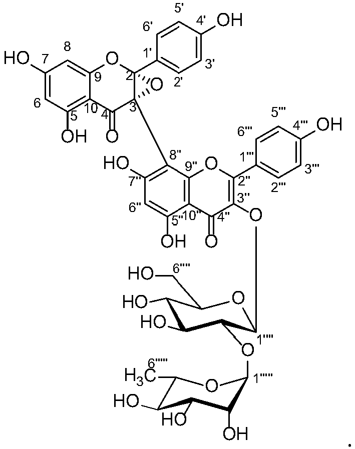

The compound was obtained as a yellow oil, soluble in methanol. Using dichloromethane/methanol (4:1, v/v) containing one drop of glacial acetic acid as the developing solvent, thin-layer chromatography was performed. Under a 254 nm UV lamp, a dark spot was observed. After color development with a solution of p-anisaldehyde and concentrated sulfuric acid followed by heating, a yellow spot was observed, suggesting a flavonoid compound; the R*_{f}* value was 0.38. The 1D and 2D NMR spectra of this compound were essentially consistent with those of Thesiuside A, confirming that they shared the same planar structure and were a pair of diastereomers. The only difference between the two compounds lay in the absolute configurations at C-2 and C-3. The ECD spectrum of this compound showed a negative Cotton effect at 320 nm and a positive Cotton effect at 288 nm, which was opposite to that observed for Thesiuside A, indicating that the absolute configurations at C-2 and C-3 were opposite between the two compounds. In summary, the structure of this compound was [(2*R*,3*R*)-2,3-epoxy-5,7,4'-trihydroxyflavanone]-(3→8)-kaempferol3"-*O*-*α*-L- rhamnopyranosyl-(1→2)-*β*-D-glucopyranoside, and it was named Thesiuside B.

The NMR spectral data for Thesiuside A and Thesiuside B are assigned as shown in the table below:

**Table 1 ¹H (600 MHz) and ¹³C (150 MHz) data for Thesiuside A and Thesiuside B (CD₃OD)**

| No. | Thesiuside A | | Thesiuside B | |
|---|---|---|---|---|
| position | *δ*_{C} | *δ*_{H} | *δ*_{C} | *δ*_{H} |
| 2 | 119.5 | | 119.5 | |
| 3 | 81.7 | | 81.7 | |
| 4 | 192.5 | | 192.5 | |
| 5 | 165.5 | | 165.6 | |
| 6 | 98.0 | 5.96, d (2.1) | 98.0 | 5.92, d (2.1) |
| 7 | 169.6 | | 169.6 | |
| 8 | 96.2 | 5.90, d (2.1) | 96.2 | 5.88, d (2.1) |
| 9 | 162.6 | | 162.6 | |
| 10 | 100.3 | | 100.2 | |
| 1' | 125.2 | | 125.1 | |
| 2'/6' | 129.6 | 7.35, d (8.9) | 129.6 | 7.36 d (8.9) |
| 3'/5' | 115.8 | 6.78, d (8.9) | 115.8 | 6.78 d (8.9) |
| 4' | 160.2 | | 160.2 | |
| 2" | 158.8 | | 158.6 | |
| 3" | 134.7 | | 134.7 | |
| 4" | 179.6 | | 179.7 | |
| 5" | 166.6 | | 166.6 | |
| 6" | 96.3 | 6.57, s | 96.2 | 6.57, s |
| 7" | 167.1 | | 167.1 | |
| 8" | 108.1 | | 108.1 | |
| 9" | 153.5 | | 153.4 | |
| 10" | 107.4^{b} | | 107.3^{b} | |
| 1‴ | 122.5 | | 122.5 | |
| 2‴/6‴ | 132.9 | 8.12, d (9.0) | 132.9 | 8.20, d (9.0) |
| 3‴/5‴ | 116.0 | 6.90, d (9.0) | 115.9 | 6.91, d (9.0) |
| 4‴ | 161.7 | | 161.7 | |
| 1ʺʺ(Glc) | 100.1 | 5.74, d (7.7) | 100.1 | 5.80, d (7.7) |
| 2ʺʺ | 79.7 | 3.63, dd (9.2, 7.7) | 80.27 | 3.63, dd (9.2, 7.7) |
| 3ʺʺ | 79.0 | 3.55, dd (9.2, 8.7) | 78.9 | 3.53, dd (9.2, 8.7) |
| 4ʺʺ | 71.9 | 3.27, dd (9.8, 8.7) | 71.8 | 3.24, dd (9.8, 8.7) |
| 5ʺʺ | 78.5 | 3.23, ddd (9.8, 5.9, 2.1) | 78.6 | 3.19, ddd (9.8, 5.9, 2.1) |
| 6ʺʺa | 62.7 | 3.73, d (12.0, 2.1) | 62.5 | 3.70, dd (12.1, 2.1) |
| 6ʺʺb | | 3.49, dd (12.0, 5.9) | | 3.43, dd (12.1, 5.9) |
| 1‴ʺ(Rha) | 102.5 | 5.23, d (1.9) | 102.8 | 5.22, d (1.9) |
| 2‴ʺ | 72.4 | 3.98, dd (3.4, 1.9) | 72.4 | 4.00, dd (3.4, 1.9) |
| 3‴ʺ | 72.3 | 3.73, dd (9.5, 3.4) | 72.3 | 3.76, dd (9.5, 3.4) |
| 4‴ʺ | 74.0 | 3.33, overlap | 74.0 | 3.33, overlap |
| 5‴ʺ | 69.9 | 3.98, overlap | 69.9 | 4.01, overlap |
| 6‴ʺ | 17.5 | 0.91, d (6.2) | 17.5 | 0.93, d (6.2) |

### (3) Structural identification of Thesiumine A

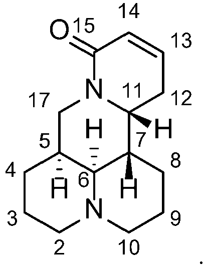

The compound was obtained as a colorless solid, soluble in methanol. Using petroleum ether/ethyl acetate (1:1, v/v) containing one drop of glacial acetic acid as the developing solvent, thin-layer chromatography was performed. Under a 254 nm UV lamp, a dark spot was observed. After color development with a modified potassium bismuth iodide reagent, an orange-red spot was observed, with an R*_{f}* value of 0.34. HRESIMS revealed a [M+H]⁺ peak at *m*/*z* 247.1805 (calculated value: 247.1805), leading to the molecular formula C₁₅H₂₂N₂O and a degree of unsaturation of 6. The ¹H NMR (600 MHz, CD₃OD) spectrum revealed sixteen proton signals from eight methylene groups: *δ*_{H} 3.46 (¹H, overlap) and 2.89 (¹H, overlap); 1.97 (¹H, overlap) and 1.72 (¹H, overlap); 1.85 (¹H, m) and 1.80 (¹H, overlap); 2.01 (¹H, overlap) and 1.72 (¹H, overlap); 2.07 (¹H, overlap) and 1.72 (¹H, overlap); 3.46 (¹H, overlap) and 2.89 (¹H, overlap); 2.79 (¹H, m) and 2.34 (¹H, m), as well as 4.25 (¹H, dd, *J* = 14.0, 5.0 Hz) and 3.23 (¹H, dd, *J* = 14.0, 12.9 Hz), four methine proton signals *δ*_{H} 4.13 (¹H, ddd, *J* = 11.8, 8.4, 7.3 Hz), 3.39 (¹H, m), 2.11 (¹H, overlap) and 2.07 (¹H, overlap), and two olefinic proton signals *δ*_{H} 6.64 (¹H, ddd, *J* = 9.8, 4.7, 3.8 Hz) and 5.84 (¹H, dt, *J* = 9.8, 2.0 Hz). The ¹³C NMR spectrum (150 MHz, CD₃OD) and the DEFT spectrum showed 15 carbon signals, including eight methylene carbon signals at *δ*_{C} 57.0, 56.9, 42.0, 28.0, 26.8, 25.2, 20.2, and 19.8, four methine carbon signals at *δ*_{C} 64.6, 52.0, 41.2, and 34.8; two olefinic carbon signals at *δ*_{C} 140.7 and 124.1; and one carbonyl signal at *δ*_{C} 167.6. The correlations of H-14/H-13/H₂-12/H-11 in the ¹H-¹H COSY spectrum, combined with the H-14/C-15, H-13/C-15, and H-11/C-13 and C-15, confirmed the positions of the α and β unsaturated ketones. The correlations of H-11/H-7/H-6/H-5/H₂-17, H-7/H₂-8/H₂-9/H₂-10, and H-5/H₂-4/H₂-3/H₂-2 in the ¹H-¹H COSY spectrum, combined with the HMBC correlations of H-11/C-15; H-17/C-15, H-17/C-11, H₂-2/C-6; H₂-10/C-6, and H₂-2/C-10, established that the basic skeleton of this compound was a matrine-type alkaloid, with a planar structure identical to that of sophocarpine. The NOESY spectrum showed correlations of H-17b/H-7 and H-11, H-3b/H-5 and H-6, H-6/H-8b, and H-11/H-8a, indicating that H-7 and H-11 were in the β-configuration, while H-5 and H-6 were in the α-configuration, thus determining the relative configuration of the compound. Based on this relative configuration, the compound was identified as a new compound.

The C-5, C-6, C-7, and C-11 positions of this compound were all chiral carbon atoms, and its absolute configuration may have two possibilities: 5R, 6R, 7R, 11S and 5S, 6S, 7S, 11R. Using computational ECD methods, ECD spectra for both configurations were obtained. It was found that the ECD curve calculated for the 5S, 6S, 7S, 11R configuration closely matched the experimentally measured ECD curve of the compound. Therefore, its absolute configuration was determined as 5S, 6S, 7S, 11R, and it was named Thesiumine A.

### (4) Structural identification of Thesiumine B

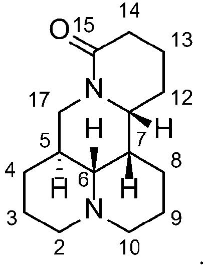

The compound was obtained as a colorless solid, soluble in methanol. Using petroleum ether/ethyl acetate (1:1, v/v) containing one drop of glacial acetic acid as the developing solvent, thin-layer chromatography was performed. Under a 254 nm UV lamp, no dark spot was observed. After color development with a modified potassium bismuth iodide reagent, an orange-red spot was observed, with an R*_{f}* value of 0.33. HRESIMS showed a [M+H]⁺ peak at *m*/*z* 249.1960 (calculated value: 249.1961), yielding a molecular formula of C₁₅H₂₄N₂O and a degree of unsaturation of 5. This compound was structurally similar to Thesiumine A. The main difference in the 1D NMR data lies in the absence of double bonds at the C-13 and C-14 positions. Its skeleton was also a matrine-type alkaloid, as confirmed by the ¹H-¹H COSY and HMBC spectra. Correlated signals in the NOESY spectrum between H-17b/H-6, H-7 and H-11, H-2b/H-6, and H-2a/H-5 indicated that H-11, H-7, and H-6 were in the β-configuration, while H-5 was in the α-configuration, thereby determining the relative configuration of the compound. Since two possible absolute configurations existed, ECD spectra for the two enantiomers, 5S, 6R, 7S, 11R and 5R, 6S, 7R, 11S, were calculated using the same method as for the compound Thesiumine A. The results indicated that the ECD curve of the 5S, 6R, 7S, 11R configuration was in good agreement with the experimentally measured ECD curve of the compound, confirming the absolute configuration of this compound as 5S, 6R, 7S, 11R. This compound was named Thesiumine B.

The NMR spectral data for Thesiumine A and Thesiumine B are summarized in the table below:

**Table 2 ¹H (600 MHz) and ¹³C (150 MHz) data for Thesiumine A and Thesiumine B (CD₃OD)**

| No. | Thesiumine A | | Thesiumine B | |
|---|---|---|---|---|
| position | *δ*_{C} | *δ*_{H} | *δ*_{C} | *δ*_{H} |
| 2a | 57.0 | 3.46, overlap | 56.9 | 3.47, overlap |
| 2b | | 2.89, overlap | | 2.88, overlap |
| 3a | 19.9 | 1.97, overlap | 19.9 | 2.01, overlap |
| 3b | | 1.70, overlap | | 1.72, overlap |
| 4a | 26.8 | 1.85, m | 26.8 | 1.86, overlap |
| 4b | | 1.80, overlap | | 1.78, overlap |
| 5 | 34.8 | 2.11, overlap | 35.5 | 1.99, overlap |
| 6 | 64.6 | 3.39, t (3.2) | 64.9 | 3.36, t (2.9) |
| 7 | 41.2 | 2.07, overlap | 43.0 | 1.84, overlap |
| 8a | 25.2 | 2.01, overlap | 25.2 | 2.06, overlap |
| 8b | | 1.72, overlap | | 1.70, overlap |
| 9a | 20.3 | 2.07, overlap | 20.3 | 2.06, overlap |
| 9b | | 1.74, overlap | | 1.70, overlap |
| 10a | 56.9 | 3.46, overlap | 56.9 | 3.47, overlap |
| 10b | | 2.89, overlap | | 2.88, overlap |
| 11 | 52.0 | 4.13, ddd, (11.8, 8.4, 7.3) | 53.7 | 4.01, ddd (11.3, 8.9, 6.0) |
| 12a | 28.0 | 2.79, m | 28.1 | 2.22, m |
| 12b | | 2.34, m | | 1.50, m |
| 13a | 140.7 | 6.64, ddd (9.8, 4.7, 3.8) | 19.3 | 1.84, overlap |
| 13b | | | | 1.72, overlap |
| 14a | 124.1 | 5.84, dt (9.8, 2.0) | 33.4 | 2.39, m |
| 14b | | | | 2.28, m |
| 15 | 167.6 | | 172.3 | |
| 17a | 42.0 | 4.25, dd (14.0, 5.0) | 41.5 | 4.49, dd (14.0, 4.7) |
| 17b | | 3.23, dd (14.0, 12.9) | | 3.18, t (14.0, 12.8) |

### (5) Spectral data for kaempferol-3-O-glucorhamnoside

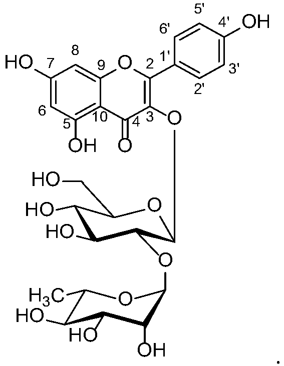

¹H NMR (600 MHz, CD₃OD): *δ* 8.04 (d, *J* = 9.0 Hz, 2H, H-2', 6'), 6.89 (d, *J* = 9.0 Hz, 2H, H-3', 5'), 6.37 (d, *J* = 2.1 Hz, ¹H, H-8), 6.17 (d, *J* = 2.1 Hz, ¹H, H-6), 5.73 (d, *J* = 7.6 Hz, ¹H, H-G₁), 5.24 (d, 1.4 Hz, ¹H, H-R₁), 4.05 (dd, *J =* 9.6, 6.2 Hz, ¹H, H-R₅), 4.01 (dd, *J* = 3.4, 1.4 Hz,¹H, H-R₂), 3.79 (dd, *J* = 9.6, 3.4 Hz, ¹H, H-R₃), 3.73 (dd, *J* = 12.1, 2.4 Hz, ¹H, H-G₆ₐ), 3.62 (dd, *J* = 9.2, 7.6 Hz, ¹H, H-G₂), 3.57 (dd, *J* = 9.3, 7.6 Hz, ¹H, H-G₃), 3.51 (dd, *J* = 12.1, 5.6 Hz, ¹H, H-G_{6b}), 3.35 (t, *J* = 9.6 Hz, ¹H, H-R₄), 3.29 (m, ¹H, H-G₄), 3.24 (m, ¹H, H-G₅), 0.97 (d,*J* = 6.2 Hz, 3H, H₃-R₆). ¹³C NMR (150 MHz, CD₃OD): *δ* 179.4 (C-4), 165.6 (C-7), 163.1 (C-5), 161.2 (C-2), 158.6 (C-4'), 158.4 (C-9), 134.5 (C-3), 132.1 (C-2', 6'), 123.2 (C-1'), 116.1 (C-3', 5'), 106.0 (C-10), 102.6 (C-R₁), 100.3 (C-G₁), 99.7 (C-6), 94.6 (C-8), 80.0 (C-G₂), 78.9 (C-G₃), 78.2 (C-G₅), 74.1 (C-R₄), 72.4 (C-R₂), 72.3 (C-R₃), 71.9 (C-G₄), 69.9 (C-R₅), 62.7 (C-G₆), 17.5 (C-R₆).

### (6) Spectral data for astragalin

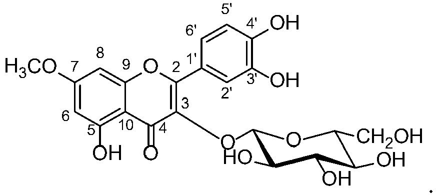

¹H NMR (600 MHz, CD₃OD): *δ* 8.05 (d, *J =* 8.9 Hz, 2H, H-2', 6'), 6.87 (d, J= 8.9 Hz, 2H, H-3', 5'), 6.39 (d, *J* = 1.6 Hz, ¹H, H-8), 6.20 (d, *J* = 1.6 Hz, ¹H, H-6), 5.25 (d, *J* = 7.5 Hz, ¹H, H-G₁), 3.69 (dd, *J* = 11.9, 2.5 Hz, ¹H, H-G₆ₐ), 3.53 (dd, *J* = 11.9, 5.5, Hz, ¹H, H-G₂), 3.41 (m, 2H, H-G₃, G_{6b}), 3.30 (m, ¹H, H-G₅), 3.20 (m, ¹H, H-G₄). ¹³C NMR (150 MHz, CD₃OD): *δ* 179.5 (C-4), 166.5 (C-7), 163.0 (C-5), 161.6 (C-4'), 159.1 (C-2), 158.6 (C-9), 135.5 (C-3), 132.3 (C-2', 6'), 122.9 (C-1'), 116.1 (C-3', 5'), 105.6 (C-10), 104.3 (C-6), 100.1 (C-G₁), 94.9 (C-8), 78.4 (C-G₂), 78.1 (C-G₅), 75.8 (C-G₃), 71.4 (C-G₄), 62.7 (C-G₆).

### (7) Spectral data for kaempferol

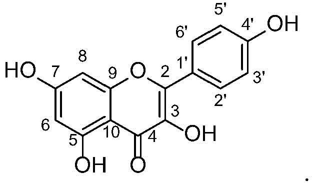

¹H NMR (600 MHz, CD₃OD): *δ* 8.10 (d, *J* = 9.0 Hz, 2H, H-2', 6'), 6.93 (d, *J* = 9.0 Hz, 2H, H-3', 5'), 6.41 (d, *J* = 2.1 Hz, ¹H, H-8), 6.21 (d, *J* = 2.1 Hz, ¹H, H-6). ¹³C NMR (150 MHz, CD₃OD): *δ* 177.4 (C-4), 165.6 (C-5), 162.5 (C-7), 160.5 (C-4'), 158.3 (C-9), 148.1 (C-2), 137.1 (C-3), 130.7 (C-2', 6'), 123.8 (C-1'), 116.3 (C-3', 5'), 104.6 (C-10), 99.3 (C-6), 94.5 (C-8).

### Example 3

The pharmaceutical composition of the present application was prepared by uniformly mixing 66.0 g of Bairuisu components and 3.0 g of matrine components. The Bairuisu components included 27.0 g of kaempferol-3-O-glucorhamnoside, 33.0 g of astragalin, 5.0 g of kaempferol, 0.5 g of Thesiuside A, and 0.5 g of Thesiuside B. The matrine components included 1.5 g of Thesiumine A and 1.5 g of Thesiumine B. Thesiuside A, Thesiuside B, Thesiumine A, and Thesiumine B were all prepared as described in Example 1.

### Example 4

The pharmaceutical composition of the present application was prepared by uniformly mixing 60.0 g of Bairuisu components and 5.0 g of matrine components. The Bairuisu components included 30.0 g of Thesiuside A and 30.0 g of Thesiuside B. The matrine components included 2.5 g of Thesiumine A and 2.5 g of Thesiumine B. Thesiuside A, Thesiuside B, Thesiumine A, and Thesiumine B were all prepared according to Example 1.

### Example 5

The pharmaceutical composition of the present application was prepared by uniformly mixing 65.0 g of Bairuisu components and 5.0 g of matrine components. The Bairuisu components included 27.0 g of kaempferol-3-O-glucorhamnoside, 33.0 g of astragalin, and 5.0 g of kaempferol. The matrine components included 2.0 g of matrine, 2.0 g of sophocarpine, 0.5 g of Thesiumine A, and 0.5 g of Thesiumine B. Both Thesiumine A and Thesiumine B were prepared according to Example 1.

### Example 6

The pharmaceutical composition of the present application was prepared by uniformly mixing 60.0 g of Bairuisu components and 20.0 g of matrine components. The Bairuisu components included 30.0 g of kaempferol-3-O-glucorhamnoside and 30.0 g of astragalin. The matrine components included 10.0 g of matrine and 10.0 g of sophocarpine.

### Example 7

The pharmaceutical composition of the present application was prepared by uniformly mixing 60.0 g of Bairuisu components and 60.0 g of matrine components. The Bairuisu components included 30.0 g of kaempferol-3-O-glucorhamnoside and 30.0 g of astragalin. The matrine components included 30.0 g of matrine and 30.0 g of sophocarpine.

### Example 8

The pharmaceutical composition of the present application was prepared by uniformly mixing 60.0 g of Bairuisu components and 5.0 g of matrine components. The Bairuisu components included 60.0 g of kaempferol. The matrine component included 5.0 g of matrine.

### Performance Testing:

### Evaluation of the Anti-inflammatory Effects of Different Pharmaceutical Compositions

### (1) Experimental Methods

RAW 264.7 cells (2 × 10⁴ cells/well) were inoculated into a 96-well plate and incubated overnight. After the cells adhered to the wall, the original medium was removed. The control group received 200 µL of DMEM medium, the model group received 200 µL of DMEM medium containing LPS (1 µg/mL), and the drug-treatment groups were exposed to 200 µL of DMEM medium containing LPS (1 µg/mL) and the test drugs. After 24 hrs of incubation, 100 µL of the supernatant from each well was taken and mixed with 100 µL of Griess reagent in a separate 96-well plate. After 15 min, the absorbance of the solution was measured at 570 nm. The NO concentration was calculated based on a standard curve prepared with NaNO₂.

The MTT assay was performed in the original 96-well plate. 20 µL of MTT solution (2 mg/mL) was added to each well. After an additional 3 hrs of incubation, the supernatant was aspirated, and 100 µL of dimethyl sulfoxide (DMSO) was added to each well. After 5 min, cell viability was determined by measuring the absorbance at 570 nm.

### (2) Experimental Results

All pharmaceutical compositions exhibited no cytotoxicity within the dose range of 2-32 µg/mL and were capable of dose-dependently inhibiting NO production. At a dose of 32 µg/mL, the maximum inhibition rates were 79.02% (the composition of Example 3, part A of FIG. 10), 89.39% (the composition of Example 4, part B of FIG. 10), 77.02% (the composition of Example 5, part C of FIG. 10), 63.86% (the composition of Example 6, part D of FIG. 10), 51.49% (the composition of Example 7, part E of FIG. 10), and 86.88% (the composition of Example 8, part F of FIG. 10), respectively. The compositions of Example 4 and Example 8 exhibited superior anti-inflammatory effects.

### Inhibitory Effects of Bairuisu Components on Nitric Oxide (NO) Production

### (1) Experimental Methods

RAW 264.7 cells (2 × 10⁴ cells/well) were inoculated into a 96-well plate for overnight incubation. After the cells adhered to the wall, the original medium was removed. The control group received 200 µL of DMEM medium, the model group received 200 µL of DMEM medium containing LPS (1 µg/mL), and the drug-treatment groups were exposed to 200 µL of DMEM medium containing LPS (1 µg/mL) and the test drugs. After 24 hrs of incubation, 100 µL of the supernatant from each well was taken and mixed with 100 µL of Griess reagent in a separate 96-well plate. After 15 min, the absorbance of the solution was measured at 570 nm. The NO concentration was calculated based on a standard curve prepared with NaNO₂.

The MTT assay was performed in the original 96-well plate. 20 µL of MTT solution (2 mg/mL) was added to each well. After an additional 3 hrs of incubation, the supernatant was aspirated, and 100 µL of dimethyl sulfoxide (DMSO) was added to each well. After 5 min, cell viability was determined by measuring the absorbance at 570 nm.

### (2) Experimental Results

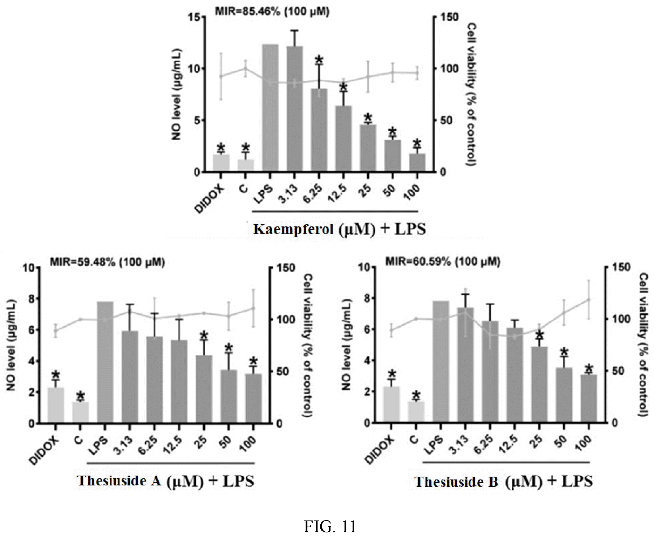

The compounds kaempferol, Thesiuside A, and Thesiuside B exhibited no cytotoxicity within the concentration range of 3.13-100 µM and were capable of dose-dependently inhibiting NO production. At a concentration of 100 µM, the maximum inhibition rates were 85.46% (kaempferol), 59.48% (Thesiuside A), and 60.59% (Thesiuside B), respectively (FIG. 11).

### Quinone Reductase (QR)-Inducing Activity of Bairuisu Components

### (1) Experimental Methods

Hepa 1c1c7 cells (2 × 10⁴ cells/well) were inoculated into a 96-well plate for overnight incubation. After the cells adhered to the wall, the original medium was removed. The control group was treated with 200 µL of DMEM medium, and the treatment groups were treated with 200 µL of DMEM medium containing different concentrations of the test compounds. After 24 hrs of incubation, the medium was removed, and 30 µL of cell lysis buffer was added to each well. After 15 min of incubation, 170 µL of the complete reaction mixture was added to each well. After 4 min, the absorbance of the solution was measured at 630 nm.

### (2) Experimental Results

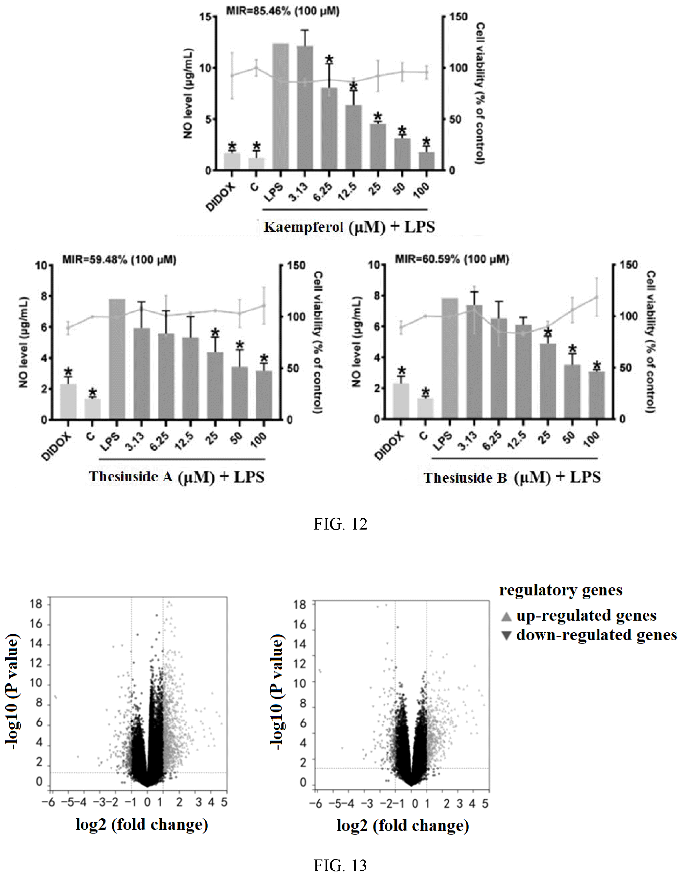

The compounds kaempferol, Thesiuside A, and Thesiuside B all induced QR activity in a dose-dependent manner. At a concentration of 100 µM, the induction factor for kaempferol reached 3.65. At the same concentration (100 µM), the maximum induction factors for Thesiuside A and Thesiuside B were 3.65 and 2.13, respectively (FIG. 12).

### Effects of Bairuisu Components and Matrine Components on the Transcriptome of Human Bronchial Epithelial Beas-2B Cells

### (1) Experimental Methods

An LPS-induced inflammatory model was established in Beas-2B cells. The samples were divided into a control group, an inflammation group, a Thesiuside A group, and a Thesiumine A group. Transcriptomic analysis was performed using an Illumina NovaSeq 6000 sequencer with a PE150 sequencing mode. After the raw data were obtained, differentially expressed genes were analyzed using the R programming language.

### (2) Experimental Results

Using |logFC| > 1 as the screening criterion, compared with the control group, the inflammation group exhibited 1,782 upregulated genes and 875 downregulated genes (FIG. 13A, FIG. 14A). Compared with the inflammation group, the Thesiuside A group exhibited 303 upregulated genes and 76 downregulated genes (FIG. 13B); the Thesiumine A group exhibited 243 upregulated genes and 92 downregulated genes (FIG. 14B). Using the Venny 2.1.0 website, the intersection of the upregulated differentially expressed genes in the inflammation group and the downregulated differentially expressed genes in the treatment group, as well as the downregulated differentially expressed genes in the inflammation group and the upregulated differentially expressed genes in the treatment group, was calculated. This yielded 379 potential anti-inflammatory functional genes (targets) for Thesiuside A and 335 potential anti-inflammatory functional genes (targets) for Thesiumine A. The intersection between the two lists contained only 42 genes (FIG. 15), indicating that there were significant differences in the inflammation-regulating genes between the two components and that the anti-inflammatory mechanisms of the two classes of components were different.

### Efficacy of Bairuisu Component and Matrine Component in Treating Pulmonary Inflammatory Diseases and Their Synergistic Mechanisms

In this study, it was confirmed that the Bairuisu component and the matrine component had significant therapeutic effects on pulmonary inflammation, and their mechanisms were further investigated. The results indicated that the Bairuisu component and the matrine component could inhibit the increase in mRNA levels of NLRP3, caspase-1, IL-1β, and COX-2, thereby suppressing inflammatory responses and oxidative stress. Meanwhile, a novel mechanism was discovered by which the Bairuisu component treated acute lung injury through regulating ferroptosis-related pathway proteins SLC7A11 and GPX4. The matrine component was found to inhibit the excessive activation of the NLRP3 inflammasome and suppress NF-κB activation. Furthermore, the Bairuisu component and the matrine component exerted regulatory effects on different gut microbiota: specifically, the Bairuisu component primarily increased the abundance of Lactobacillus murinus, while the matrine component primarily increased the abundance of *Bacteroides caecimuris* and *Bacteroides vulgatus*. It was also found that changes in these bacterial strains were correlated with the expression of immune factors. The two components synergistically suppressed inflammatory responses and oxidative stress, thereby inhibiting the progression of pulmonary inflammatory diseases.

### 1. Efficacy of Bairuisu Component and Matrine Component in Treating Acute Lung Injury and Their Synergistic Mechanisms

### (1) Experimental Methods

Male C57BL/6N mice (aged 6-7 weeks, weighing 19-21 g) were purchased from Beijing Vital River Laboratory Animal Technology Co., Ltd. They were housed in a temperature- and humidity-controlled animal facility under a 12 hrs light/dark cycle with free access to food and water.

Animal grouping: Fifty mice were randomly divided into 10 groups (n = 5): the LPS (10 mg/kg) group, the control group (CTRL), the positive control drug (DEX, 1 mg/kg) group, the kaempferol-3-O-glucorhamnoside (BS1, 20 mg/kg) group, the astragalin (BS2, 20 mg/kg) group, the kaempferol (BS3, 20 mg/kg) group, the matrine (MR, 20 mg/kg) group, and the combination (BS3 + MR, kaempferol 20 mg/kg + matrine 20 mg/kg) group.

Model induction and drug administration: Using a liquid aerosol pulmonary delivery device, 10 mg/kg of LPS (dissolved in saline) was instilled into the trachea of the mice. Drug administration was carried out via intragastric gavage 4 hrs before model induction; all drugs were dissolved in a 5% ethanol saline solution. Drug administration and model induction were performed every other day, and the mice were sacrificed on day 14.

Complete blood count (CBC): Twenty-four hours after the last drug administration, peripheral blood was collected from the mice via orbital puncture and placed into 5 mL EDTA-K₂ anticoagulant tubes. A Mindray BC-6800 automatic blood cell analyzer was used to perform the CBC.

Collection of bronchoalveolar lavage fluid (BALF) and ELISA assay: After the mice were sacrificed by cervical dislocation, the trachea was exposed by dissection, and 1 mL of PBS was injected into the trachea using a syringe. The lavage fluid was recovered after 45 s, and the procedure was repeated three times. The collected fluid was centrifuged at 2000 rpm for 10 min, and the supernatant was collected as BALF and stored at -20°C. The BALF was analyzed using an enzyme-linked immunosorbent assay (ELISA).

Histopathological evaluation: The entire right lower lobe of the freshly excised lung was immersed in 4% paraformaldehyde for 24 h, dehydrated, and embedded in paraffin. The lung tissues were sectioned into 4 µm thick sections and dewaxed: xylene I (16 min), xylene II (16 min), anhydrous ethanol I (6 min), anhydrous ethanol II (6 min), 75% ethanol (6 min), and washed three times with water. After dewaxing, the sections were stained with hematoxylin and eosin (H&E).

Lung tissue homogenate protein analysis: Tissue proteins were collected, denatured, and sonicated. After centrifugation, the cell lysate supernatant was collected for SDS-PAGE analysis, followed by membrane transfer, blocking, and incubation with the corresponding antibody overnight at 4°C. The membrane was washed with PBST, and a horseradish peroxidase-conjugated secondary antibody was added for 1 hrs of incubation to analyze protein levels.

qRT-PCR for detection of inflammatory factor mRNA levels: Mouse lung tissue was minced, and total mRNA was extracted using Trizol reagent. After reverse transcription, the mRNA levels of NLRP3, caspase-1, IL-1β, COX-2, SLC7A11, and GPX4 were detected by qRT-PCR.

Statistical analysis: Data were analyzed using GraphPad Prism 8.0 software. One-way analysis of variance (ANOVA) and the LSD test were used for multiple comparisons. A statistically significant difference was established at P < 0.05 or P < 0.01. Data were presented as means ± SD.

### (2) Experimental Results

Both the Bairuisu components and the matrine component attenuated LPS-induced pathological lung damage in mice. The lung tissue structure in the control group was intact, with no hemorrhage or edema. In contrast, the LPS group showed persistent infiltration of inflammatory cells, accumulation of neutrophils in the alveolar and interstitial spaces, thickening of the alveolar and airway walls, and severe hemorrhage. The kaempferol-3-O-glucorhamnoside (BS1), astragalin (BS2), kaempferol (BS3), matrine (MR), and combination treatment groups all significantly alleviated these inflammatory symptoms, with the combination group showing a better effect (FIG. 16).

Both the Bairuisu components and the matrine component inhibited LPS-induced production of the inflammatory cytokine IL-1β in mouse lungs. The level of IL-1β in the BALF of mice in the LPS model group was significantly elevated, while treatment with kaempferol-3-O-glucorhamnoside (BS1), astragalin (BS2), kaempferol (BS3), matrine (MR), and the combination reduced the level of IL-1β, and the combined administration of kaempferol and matrine showed better effects than either agent alone (FIG. 17).

Both the Bairuisu components and the matrine component reduced the number of inflammatory cells in the peripheral blood of LPS-induced mice. The white blood cell and neutrophil counts in the blood of mice in the LPS model group were significantly increased, indicating that LPS induced systemic inflammation in the mice. Treatment with kaempferol-3-O-glucorhamnoside (BS1), astragalin (BS2), kaempferol (BS3), matrine (MR), and the combination reduced both counts. Therefore, both the Bairuisu components and the matrine component reduced the systemic inflammatory response (FIG. 18).

Both the Bairuisu components and the matrine component inhibited the LPS-induced increase in the mRNA levels of inflammatory factors in mouse lung tissue. The qRT-PCR results showed that kaempferol-3-O-glucorhamnoside (BS1), astragalin (BS2), kaempferol (BS3), matrine (MR), and the combination inhibited the increase in the mRNA levels of NLRP3, caspase-1, IL-1β, and COX-2 (FIG. 19).

The Bairuisu components exerted a lung-protective effect by regulating ferroptosis-related pathways. In the LPS model group, the levels of the ferroptosis-related pathway proteins SLC7A11 and GPX4 were significantly decreased, while administration of kaempferol-3-O-glucorhamnoside (BS1), astragalin (BS2), and kaempferol (BS3) significantly promoted the increase in SLC7A11 and GPX4 (FIG. 20).

### 2. Efficacy of Bairuisu Component and Matrine Component in Treating Chronic Obstructive Pulmonary Disease and Their Synergistic Mechanisms

### (1) Experimental Methods

Male C57BL/6N mice (aged 6-7 weeks, weighing 19-21 g) were purchased from Beijing Vital River Laboratory Animal Technology Co., Ltd. They were housed in a temperature- and humidity-controlled animal facility under a 12 hrs light/dark cycle with free access to food and water.

Animal grouping: Twenty-five mice were randomly divided into five groups (n = 5): a control (CTRL) group, a cigarette smoke-induced (CS) group, a positive control drug (DEX, 1 mg/kg) group, a Bairuisu component (Thesiuside A, BSA, 20 mg/kg) group, and a matrine component (Thesiumine A, BJA, 20 mg/kg) group.

Model induction and drug administration: A cigarette smoke-induced COPD model was established. Mice were exposed to cigarette smoke daily for 16 weeks. Drug treatment was initiated at week 8. Drugs were administered 4 hrs prior to smoke exposure and were dissolved in a 5% ethanol saline solution. Cigarette smoke exposure was performed 6 days per week for 2 hrs each day, and drug treatment was administered once daily.

Complete blood count (CBC): Twenty-four hours after the last drug administration, peripheral blood was collected from the mice via orbital puncture and placed into 5 mL EDTA-K₂ anticoagulant tubes. A Mindray BC-6800 automatic blood cell analyzer was used to perform the CBC.

Collection of bronchoalveolar lavage fluid (BALF) and ELISA assay: After the mice were sacrificed by cervical dislocation, the trachea was exposed by dissection, and 1 mL of PBS was injected into the trachea using a syringe. The lavage fluid was recovered after 45 s, and the procedure was repeated three times. The collected fluid was centrifuged at 2000 rpm for 10 min, and the supernatant was collected as BALF and stored at -20°C. The BALF was analyzed using an enzyme-linked immunosorbent assay (ELISA).

Histopathological evaluation: The entire right lower lobe of the freshly excised lung was immersed in 4% paraformaldehyde for 24 h, dehydrated, and embedded in paraffin. The lung tissues were sectioned into 4 µm thick sections and dewaxed: xylene I (16 min), xylene II (16 min), anhydrous ethanol I (6 min), anhydrous ethanol II (6 min), 75% ethanol (6 min), and washed three times with water. After dewaxing, the sections were stained with hematoxylin and eosin (H&E).

Lymphocyte subtype analysis of lung tissue: Mouse lung tissue was collected, chopped, digested with collagenase, ground, and filtered. The cells were separated by PERCOLL gradient, red blood cells were lysed, and the cells were centrifuged and resuspended. Membrane antibody staining was performed, cells were stimulated for differentiation, membranes were disrupted, intracellular antibody staining was performed, and the cells were centrifuged, resuspended, and analyzed on a flow cytometer.

Mouse fecal 16S rRNA detection: Three to four mouse fecal pellets were collected into sterile EP tubes. DNA extraction and purification, PCR amplification, product purification, library preparation and quality control were performed, followed by sequencing on a NovaSeq platform.

Statistical analysis: Data were analyzed using GraphPad Prism 8.0 software. One-way analysis of variance (ANOVA) and the LSD test were used for multiple comparisons. A statistically significant difference was established at P < 0.05 or P < 0.01. Data were presented as means ± SD.

### (2) Experimental Results

Both the Bairuisu component and the matrine component attenuated CS-induced pathological damage in mouse lungs. The lung tissue structure in the control group was intact, with no hemorrhage or edema. In contrast, the CS group exhibited enlarged alveolar spaces and increased alveolar tissue damage. The Bairuisu component and the matrine component alleviated these pathological symptoms (FIG. 21).

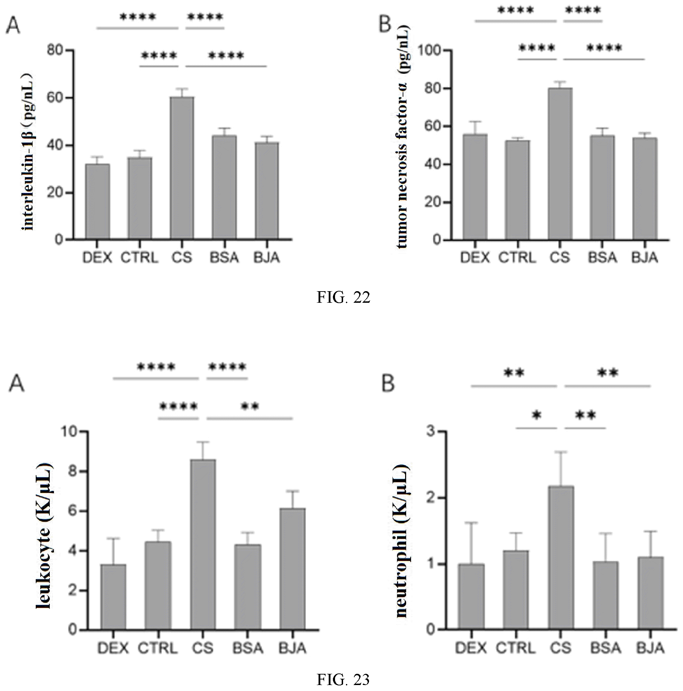

Both the Bairuisu component and the matrine component inhibited the production of the inflammatory cytokines IL-1β and TNF-α in CS-induced mouse lungs. The levels of IL-1β and TNF-α in the BALF of mice in the CS model group were significantly elevated, while treatment with the Bairuisu component and the matrine component reduced the levels of these inflammatory cytokines (FIG. 22).

Both the Bairuisu component and the matrine component reduced the number of inflammatory cells in the peripheral blood of CS-induced mice. The white blood cell and neutrophil counts in the blood of mice in the CS model group were significantly increased, indicating that CS induced systemic inflammation in the mice; treatment with the Bairuisu component and the matrine component reduced both counts. Therefore, both the Bairuisu component and the matrine component reduced the systemic inflammatory response (FIG. 23).

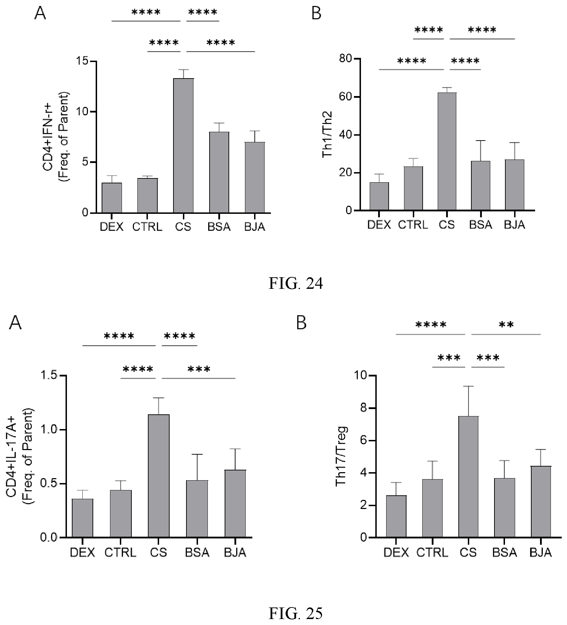

Both the Bairuisu component and the matrine component suppressed the proportions of Th1 and Th17 cells in CS-induced mouse lung tissue. In CS-treated mice, the proportions of Th1 and Th17 cells were significantly increased, leading to disruption of the balance between Th1 and Th2 as well as between Th17 and Treg, thereby triggering an immune response. The results showed that the Bairuisu component and the matrine component significantly suppressed the proportions of Th1 and Th17 cells, thereby normalizing the balance between Th1 and Th2 as well as between Th17 and Treg (FIGS. 24, 25).

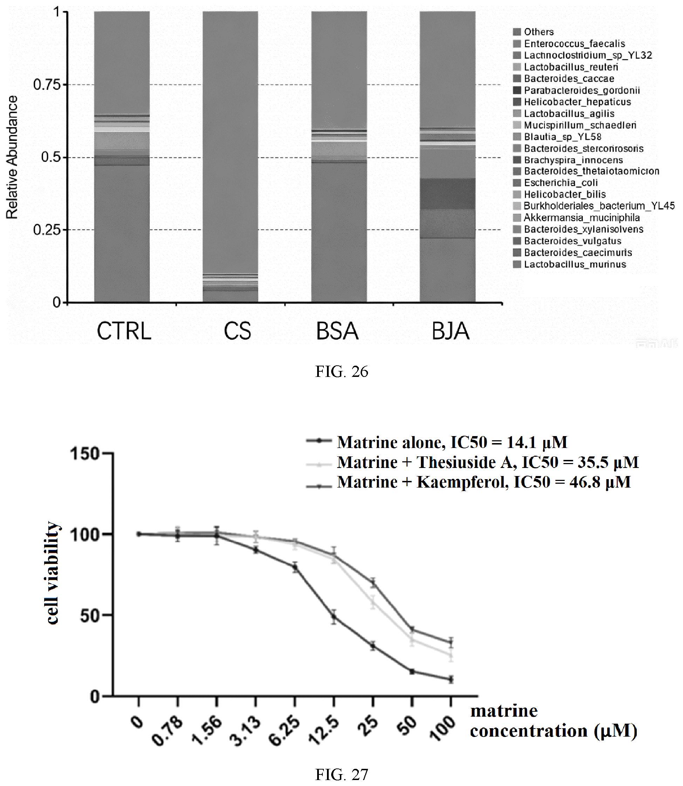

Both the Bairuisu component and the matrine component improved the composition of the gut microbiota in CS-induced COPD mice. In CS-induced mice, the gut microbiota was dysbiotic compared with the control mice; however, after intervention with the Bairuisu component and the matrine component, the composition of the gut microbiota was significantly improved. Specifically, the Bairuisu component primarily exerted its beneficial effects on COPD by increasing the abundance of *Lactobacillus murinus*, while the matrine component primarily did so by increasing the abundance of *Bacteroides caecimuris* and *Bacteroides vulgatus* (FIG. 26).

### Binding Interactions of Bairuisu Components and Matrine Component with Key Inflammatory Regulatory Proteins

### (1) Experimental Methods

The structures of the compounds were constructed using ChemDraw 20.0 software and were converted into three-dimensional structures using Chem3D. The structural coordinates of iNOS and COX-2 were retrieved in PDB format from the RCSB Protein Data Bank (www.rcsb.org; PDB IDs: 3E6T and 5IKQ). Molecular docking was performed using the Maestro 12.8 module within the Schrödinger software. The protein structures were preprocessed and optimized using the Protein Preparation Wizard. Subsequent ligand-protein molecular docking studies were carried out using Glide with the Standard Precision (SP) docking mode and default parameter settings, and the binding interactions were analyzed. The ligands were ranked against the target proteins based on the Glide scores.

### (2) Experimental Results

Representative Bairuisu components and the matrine component exhibited strong binding affinity for the key inflammatory regulatory proteins iNOS and COX-2. Among them, the new Bairuisu components Thesiuside A and Thesiuside B showed significantly superior binding affinity for iNOS and COX-2 compared to the known Bairuisu component kaempferol; the new matrine component Thesiumine B also showed superior binding affinity for iNOS compared to the known matrine components matrine and sophocarpine.

The binding affinities of representative Bairuisu components and the matrine component to the key inflammatory regulatory proteins are shown in Table 3 below:

**Table 3 Binding affinities of representative Bairuisu components and the matrine component to key inflammatory regulatory proteins**

| Compound Name | Glide score (iNOS) | Glide score (COX-2) |
|---|---|---|
| Thesiuside A | -6.641 | -8.798 |
| Thesiuside B | -7.323 | -7.305 |
| kaempferol | -5.883 | -5.295 |
| Thesiumine A | -4.185 | -5.561 |
| Thesiumine B | -5.474 | -4.514 |
| matrine | -4.567 | -4.538 |
| sophocarpine | -3.982 | -5.341 |

### Inhibition of Matrine Hepatotoxicity by Bairuisu Components

### 1. Combination of Bairuisu Component with Matrine Reduces the Hepatotoxicity of Matrine

### (1) Experimental Methods

Human liver immortalized THLE-2 cells (2 × 10⁴ cells/well) were seeded into a 96-well plate and incubated overnight. The original medium was aspirated, and medium containing matrine at test concentrations or matrine together with Bairuisu components was added. The cells were incubated for 24 hrs. The medium was then discarded, and 20 µL of MTT solution (2 mg/mL) was added to each well. After 3 hrs of incubation, the culture supernatant was aspirated, and 100 µL of dimethyl sulfoxide (DMSO) was added to each well. After 5 min, cell viability was measured by determining the absorbance at 570 nm.

### (2) Experimental Results

Matrine (MR) was toxic to normal liver cells, inhibiting cell viability in a dose-dependent manner, with a half-maximal inhibitory concentration (IC₅₀) of 14.1 µM. Coadministration with Thesiuside A (BSA) and kaempferol (BS3) effectively reduced the hepatotoxicity of matrine. Specifically, the IC₅₀ increased to 35.5 µM when MR was co-administered with BSA, and to 46.8 µM when co-administered with BS3 (FIG. 27).

### 2. Combination of Bairuisu Component with Matrine Reduces Matrine-Induced Reactive Oxygen Species Production

### (1) Experimental Methods

Human liver THLE-2 cells (2 × 10⁵ cells/well) were seeded in D35 dishes and incubated overnight. The original medium was aspirated, and medium containing matrine at test concentrations or matrine together with Bairuisu components was added. The cells were incubated for 24 hrs. The medium was then discarded, and the cells were harvested after digestion with EDTA-free trypsin and washed twice with PBS. Then, 200 µL of DCHF-DA solution (200 µg/mL) was added, and the cells were incubated for 2 hrs. The cells were washed three times with PBS, and cellular ROS levels were measured by flow cytometry.

### (2) Experimental Results

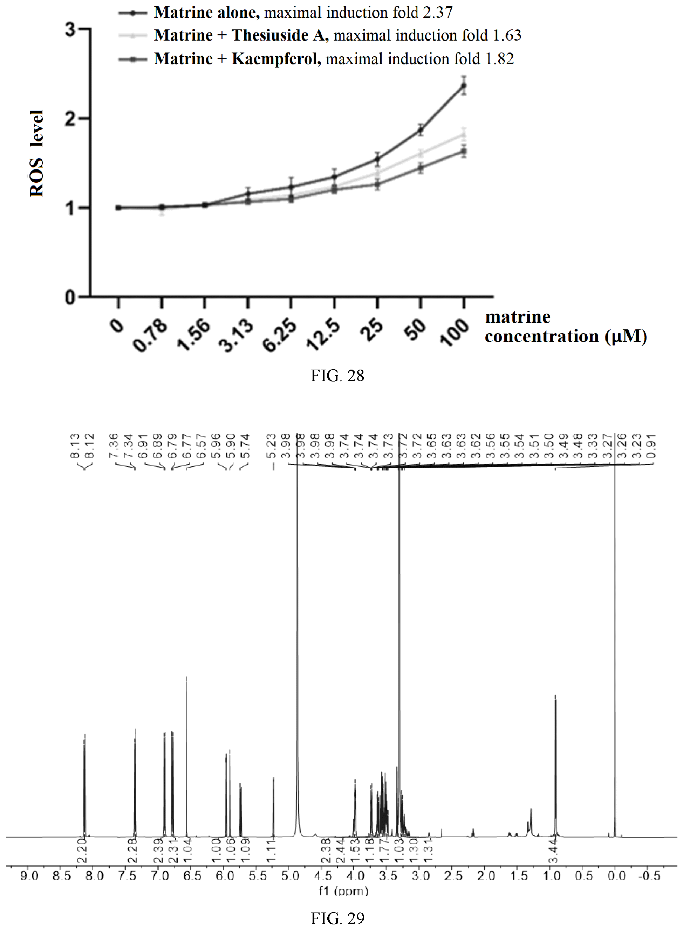

Matrine (MR) induced oxidative stress in THLE-2 cells, increasing ROS levels in a dose-dependent manner, with a maximum induction factor of 2.37-fold. Combination with Thesiuside A (BSA) and kaempferol (BS3) effectively reduced the ROS levels induced by matrine. Specifically, the maximum induction fold decreased to 1.63-fold when matrine was combined with BSA, and to 1.82-fold when combined with BS3 (FIG. 28).

### Matrine Component Enhances the Water Solubility of Bairuisu Component

### (1) Experimental Methods

An excess amount of kaempferol was weighed. 1.0 mL of distilled water was added to the blank group, and 1.0 mL of matrine aqueous solution was added to the mixed group, followed by thorough mixing. The samples were placed in an ultrasonic cleaner and sonicated for 30 min. The mixtures were then centrifuged at 8000 r·min⁻¹ for 10 min (centrifugation radius: 12 cm). The supernatants were filtered through a 0.22 µm microporous membrane and analyzed by HPLC. The peak areas were recorded; the peak area of kaempferol in the blank group was designated as A1, and that in the mixed group as A2. The fold increase in peak area was calculated according to the following formula: Fold increase in peak area = (A2 - A1) / A1.

### (2) Experimental Results

The peak area of kaempferol in the mixed group increased by 7-fold compared with that in the blank group, indicating that the matrine component could significantly enhance the water solubility of the Bairuisu component.

In summary, the pharmaceutical composition provided by the present application mainly comprises a Bairuisu component and a matrine component. The Bairuisu component has QR-inducing activity, is capable of inhibiting the excessive production of NO in LPS-induced RAW264.7 mouse macrophages, alleviating LPS-induced pathological changes in mouse lung tissue, inhibiting the LPS-induced increase of the inflammatory cytokine IL-1β in mouse bronchoalveolar lavage fluid, and reducing the number of inflammatory cells in mouse blood, thereby exerting a lung-protective effect and alleviating pulmonary inflammation. The Bairuisu component and the matrine component have a synergistic effect, and they exert therapeutic effects on pulmonary diseases through different mechanisms. Specifically, the Bairuisu component acts through a novel mechanism of regulating ferroptosis-related pathway proteins SLC7A11 and GPX4 to treat acute lung injury, while the matrine component can inhibit the excessive activation of the NLRP3 inflammasome and suppress NF-κB activation. The two components synergistically exert a lung-protective effect through different mechanisms. Moreover, the binding of the Bairuisu components and the matrine component to key inflammation-regulating proteins provides better anti-inflammatory effects. Therefore, the two components have an interaction characterized by "synergistic efficacy, reduced toxicity, complementary mechanisms, and mutual solubilization," which is conducive to widespread application.

The foregoing are merely optional embodiments of the present application and are not intended to limit the scope of the present application. For those skilled in the art, the present application may be subject to various modifications and variations. Any modifications, equivalent substitutions, improvements, etc., made within the spirit and principles of the present application should be included within the scope of the claims of the present application.

## Claims

1. A pharmaceutical composition for treating pulmonary inflammatory disease, comprising the following components:
1 to 100 parts of a Bairuisu component, and
1 to 20 parts of a matrine component;
wherein
the Bairuisu component has a structural formula as follows: 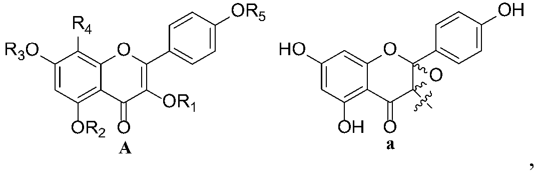
in which, R₄ represents H or group a; R₁, R₂, R₃, and R₅ are independently selected from one of the following groups: H, CH₃, CH₃CO, glucopyranosyl, rhamnopyranosyl, glucorhamnosyl, diglucosyl, xylopyranosyl, sucrosyl; and
the matrine component has a structural formula as follows: 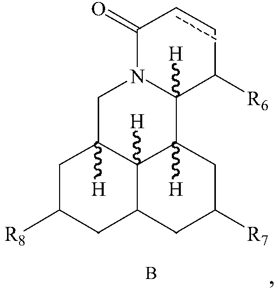
in which, R₆, R₇, and R₈ are independently selected from one of the following groups: H, OH, OCH₃, or CH₃COO.

2. The pharmaceutical composition for treating the pulmonary inflammatory disease according to claim 1, wherein the pharmaceutical composition comprises the following components:
60 to 66 parts of the Bairuisu component, and
2 to 5 parts of the matrine component.

3. The pharmaceutical composition for treating the pulmonary inflammatory disease according to claim 1, wherein the Bairuisu component is selected from at least one of kaempferol-3-O-glucorhamnoside, astragalin, kaempferol, Thesiuside A, and Thesiuside B.

4. The pharmaceutical composition for treating the pulmonary inflammatory disease according to claim 1, wherein the matrine component is selected from at least one of matrine, sophocarpine, Thesiumine A, and Thesiumine B.

5. The pharmaceutical composition for treating the pulmonary inflammatory disease according to claim 3 or 4, wherein the pharmaceutical composition comprises the following components:
10 to 20 parts of Thesiuside A,
10 to 20 parts of Thesiuside B,
1 to 2 parts of Thesiumine A, and
1 to 2 parts of Thesiumine B;
or alternatively,
the pharmaceutical composition comprises the following components:
10 to 15 parts of kaempferol, and
1 part of matrine.

6. The pharmaceutical composition for treating pulmonary inflammatory disease according to claim 3, wherein an extraction method of the Thesiuside A and the Thesiuside B comprises the following steps:
crushing whole herbs of dried *Thesium chinense* Turcz., soaking at 20°C to 40°C for 1 hr to 10 hrs, followed by extraction by heating under reflux with 60% to 80% ethanol to obtain an extract solution; filtering and concentrating the extract solution in sequence to obtain a total crude extract;
suspending the total crude extract in water, then sequentially extracting with petroleum ether, dichloromethane and ethyl acetate to obtain organic extract solutions;
subjecting an ethyl acetate extract solution to a normal-phase silica gel column chromatography, wherein a gradient elution is performed first with a petroleum ether/ethyl acetate eluent in a volume ratio of (0.1 to 1) : (0.1 to 1), and then with an ethyl acetate/methanol eluent in a volume ratio of (0.1 to 1) : (0.1 to 1); collecting eight fractions in chronological order to obtain first elution fractions E1 to E8;
subjecting a first elution fraction E7 to an MCI column to remove pigments, performing gradient elution with a methanol/water eluent in a volume ratio of (1 to 3) : ( 0.1 to 7), collecting eight fractions in chronological order to obtain second elution fractions E7a to E7h;
subjecting a second elution fraction E7d to normal-phase silica gel column chromatography, performing gradient elution with a dichloromethane/methanol eluent in a volume ratio of (0.1 to 30) : 1, collecting eight fractions in chronological order to obtain third elution fractions E7a to E7h; and
subjecting a third elution fraction E7d5 to separation and purification using Sephadex LH-20 and C18 reverse-phase silica gel to obtain the compounds Thesiuside A and Thesiuside B.
wherein
Thesiuside A has a structural formula as follows: 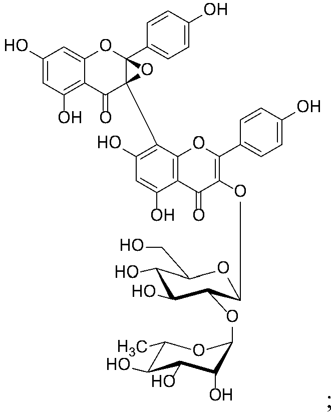 and
Thesiuside B has a structural formula as follows:

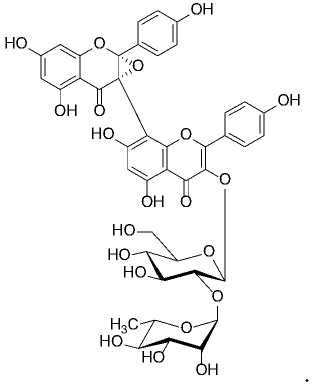

7. The pharmaceutical composition for treating the pulmonary inflammatory disease according to claim 4, wherein an extraction method of Thesiumine A and Thesiumine B comprises the following steps:
crushing whole herbs of dried *Thesium chinense* Turcz., extracting with an aqueous hydrochloric acid solution having a mass concentration of 0.1 mol/L to 0.5 mol/L, adjusting a resulting extract solution to an alkaline pH, repeatedly extracting with dichloromethane, and concentrating under reduced pressure to obtain a total alkaloid extract;
subjecting the total alkaloid extract to a normal-phase silica gel column chromatography, eluting with a petroleum ether/dichloromethane/ammonia water eluent in a volume ratio of 1: 1: (0.02 to 0.03); performing gradient elution with a dichloromethane/methanol eluent in a volume ratio of (6 to 97): (3 to 4), collecting 10 fractions in chronological order to obtain fourth elution fractions S1 to S10;
purifying a fourth elution fraction S5 by Sephadex LH-20 column chromatography using methanol as an eluent, subjecting a purified S5 fraction to HPLC separation using a mobile phase having a pH of 3 to 4 and a methanol volume percentage of 5% to 10%; and
removing impurities through a macroporous adsorption resin column to obtain Thesiumine B and Thesiumine A;
wherein
Thesiumine A has a structural formula as follows: 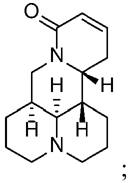 and
Thesiumine B has a structural formula as follows:

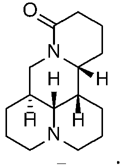

8. The pharmaceutical composition for treating the pulmonary inflammatory disease according to claim 3, wherein the extraction process for kaempferol-3-O-glucorhamnoside, astragalin, and kaempferol comprises the following steps:
crushing whole herbs of dried *Thesium chinense* Turcz., soaking at 20°C to 40°C for 1 hr to 10 hrs, followed by extraction by heating under reflux with an aqueous ethanol solution having a mass percentage of 60% to 80% to obtain an extract solution; filtering and concentrating the extract solution in sequence to obtain a total crude extract;
suspending the total crude extract in water, then sequentially extracting with petroleum ether, dichloromethane and ethyl acetate to obtain organic extract solutions;
diluting an ethyl acetate extract solution with water, passing a resulting mixture through an MCI column, and then eluting sequentially with aqueous methanol solutions having mass percentages of 20 to 30%, 40 to 50%, and 60 to 70%, yielding a first fraction, a second fraction, and a third fraction, respectively;
further eluting the first fraction sequentially with pure water, aqueous ethanol solutions having mass percentages of 20% to 30%, 40% to 50%, and 60% to 70%, and pure ethanol, yielding a fraction a, a fraction b, a fraction c, a fraction d, and a fraction e, respectively;
purifying the fraction b by gel column chromatography using methanol as an eluent, followed by recrystallization to obtain kaempferol-3-O-glucorhamnoside;
purifying the fraction c by gel column chromatography using methanol as the eluent, followed by recrystallization to obtain astragalin; and
purifying the third fraction by gel column chromatography using methanol as the eluent, and followed by recrystallization to obtain kaempferol.

9. A preparation for treating pulmonary inflammatory diseases, comprising: the pharmaceutical composition according to any one of claims 1-8 as the active ingredient, having a mass percentage in the preparation of 0.1% to 99.9%, with a remainder being pharmaceutically acceptable excipients;
wherein
a dosage form of the preparation is a tablet, sugar-coated tablet, film-coated tablet, enteric-coated tablet, capsule, oral solution, buccal tablet, granules, oral granules, pill, powder, ointment, pellet, suspension, fine powder, injection, suppository, cream, spray, drop, or patch.

10. An application of the pharmaceutical composition for treating the pulmonary inflammatory diseases according to any one of claims 1-7 in the manufacturing of a medicament for treating chronic obstructive pulmonary disease, acute lung injury, or lower respiratory tract infections.
